(19) Europäisches Patentamt / European Patent Office / Office européen des brevets

(11) **EP 4 094 775 A1**

(12) # EUROPEAN PATENT APPLICATION
published in accordance with Art. 153(4) EPC

(43) Date of publication:
**30.11.2022 Bulletin 2022/48**

(21) Application number: **21750885.2**

(22) Date of filing: **08.02.2021**

(51) International Patent Classification (IPC):
***A61K 38/43*** (2006.01)

(52) Cooperative Patent Classification (CPC):
**A61K 38/43**

(86) International application number:
**PCT/CN2021/075921**

(87) International publication number:
**WO 2021/155867 (12.08.2021 Gazette 2021/32)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **06.02.2020 CN 202010081324**

(71) Applicant: **Talengen International Limited
Hong Kong 999077 (HK)**

(72) Inventor: **LI, Jinan
Shenzhen City, Guangdong 518020 (CN)**

(74) Representative: **karo IP
karo IP Patentanwälte
Kahlhöfer Rößler Kreuels PartG mbB
Postfach 32 01 02
40416 Düsseldorf (DE)**

Remarks:
The complete document including Reference
Table(s) and the Sequence Listing(s) can be
downloaded from the EPO website

(54) **METHOD AND DRUG FOR PREVENTING AND TREATING MULTIPLE SCLEROSIS**

(57) Provided is a method for preventing and treating multiple sclerosis, comprising administering a therapeutically effective amount of a component of plasminogen activation pathway to a subject. Also provided are a medicament, a pharmaceutical composition, a product, and a kit which comprise a component of plasminogen activation pathway for treating said disease.

EP 4 094 775 A1

## Description

### TECHNICAL FIELD

The present application relates to a method for preventing or treating multiple sclerosis comprising administering to a subject an effective amount of a component of plasminogen activation pathway or a related compound thereof, such as plasminogen, to repair nerve damages and improve clinical signs and symptoms.

### BACKGROUND

Multiple sclerosis (MS) is the most common demyelinating disease of the central nervous system. Magnetic resonance imaging (MRI) reveals multiple plaque-like lesions in the brain as well as in the spinal cord. The etiology of the disease is unclear and is related to genetic factors, viral infections, and autoimmune reactions et al. There are multiple inflammatory demyelinating plaques in the white matter of the central nervous system during the acute phase of the disease, while old lesions form calcified plaques due to the proliferation of glial fibers. The disease is characterized by multiple foci, remission, and a relapsing course, and commonly occurs in the optic nerve, spinal cord, and brain stem. Current treatments, including hormonotherapy, interferon β and immunosuppressive therapy, are less than optimal and other treatments are required to be explored.

### SUMMARY OF THE APPLICATION

In the present application, it's found that plasminogen can significantly promote the regeneration and repair of nerve myelin sheath, and prevent and treat multiple sclerosis.
Specifically, the present application relates to the following:

1. In one aspect, the present application relates to a method for preventing and treating multiple sclerosis comprising administering to a subject a therapeutically effective amount of one or more compounds selected from the group consisting of: a component of plasminogen activation pathway, a compound directly activating plasminogen or indirectly activating plasminogen by activating an upstream component of a plasminogen activation pathway, a compound mimicking the activity of plasminogen or plasmin, a compound up-regulating the expression of plasminogen or activators of plasminogen, analogs of plasminogen, an analog of plasmin, an analog of tPA or uPA and an antagonist of fibrinolysis inhibitor.

In one aspect, the present application relates to the use of one or more compounds in the preparation of medicament for preventing and treating multiple sclerosis, where said one or more compounds are selected from the group consisting of: a component of a plasminogen activation pathway, a compound directly activating plasminogen or indirectly activating plasminogen by activating an upstream component of a plasminogen activation pathway, a compound mimicking the activity of plasminogen or plasmin, a compound up-regulating the expression of plasminogen or an activator of plasminogen, an analog of plasminogen, an analog of plasmin, an analog of tPA or uPA and an antagonist of fibrinolysis inhibitor.
In one aspect, the present application relates to a medicament for the prevention and treatment of multiple sclerosis comprising one or more compounds selected from the group consisting of: a component of plasminogen activation pathway, a compound directly activating plasminogen or indirectly activating plasminogen by activating an upstream component of plasminogen activation pathway, a compound mimicking the activity of plasminogen or plasmin, a compound up-regulating the expression of plasminogen or an activator of plasminogen, an analog of plasminogen, an analog of plasmin, an analog of tPA or uPA, and an antagonist of fibrinolysis inhibitor.
In one aspect, the present application relates to the use of one or more compounds in the prevention and treatment of multiple sclerosis, wherein said one or more compounds are selected from the group consisting of: a component of a plasminogen activation pathway, a compound directly activating plasminogen or indirectly activating plasminogen by activating an upstream component of a plasminogen activation pathway, a compound mimicking the activity of plasminogen or plasmin, a compound up-regulating the expression of plasminogen or an activator of plasminogen, an analog of plasminogen, an analog of plasmin, an analog of tPA or uPA, and an antagonist of fibrinolysis inhibitor.
2. The method, use, or medicament according to item 1, wherein the component of a plasminogen activation pathway is selected from the group consisting of: plasminogen, recombinant human plasmin, Lys-plasminogen, Glu-plasminogen, plasmin, a variant or an analog of plasminogen or plasmin comprising one or more kringle domains or protease domains of plasminogen and plasmin, mini-plasminogen, mini-plasmin, micro-plasminogen, micro-plasmin, delta-plasminogen, delta-plasmin, an activator of plasminogen, tPA and uPA.

3. The method, use, or medicament according to item 1, wherein the antagonist of the fibrinolysis inhibitor is an inhibitor of PAI-1, complement C1 inhibitor, α2 antiplasmin or α2 macroglobulin, e.g., an antibody.

4. The method, use, or medicament according to any one of items 1-3, wherein the compound has one or more activities selected from the group consisting of: promoting regeneration of nerve myelin sheath, promoting the expression of myelin protein (e.g., promoting the expression of PLP or MBP protein), promoting the expression of NFP in nerve tissue, promoting regeneration of nerve fiber, increasing the level of MBP in nerve tissue, increasing the number of microglia in nerve tissue, promoting repair of nerve tissue inflammation, promoting the activity of astrocyte in nerve tissue, increasing the level of BDNF in nerve tissue, promoting the expression of GFAP in nerve tissue, improving the social behavior ability of the subject, improving the social memory ability of the subject, alleviating the depressive behavior of the subject, alleviating the anxious behavior of the subject.

5. The method, use, or medicament according to any one of items 1-4, wherein the plasminogen alleviates psychiatric symptoms of the subject, such as depressive symptoms or anxiety symptoms.

6. The method, use, or medicament according to any one of items 1-5, wherein the compound is plasminogen (also known as fibrinogen).

7. The method, use, or medicament according to any one of items 1-6, wherein the plasminogen has at least 75%, 80%, 85%, 90%, 95%, 96%, 97%, 98%, or 99% sequence identity with SEQ ID NO: 2 and still has plasminogen activity, such as proteolytic activity, lysine binding activity, or both proteolytic activity and lysine binding activity.

8. The method, use, or medicament according to any one of items 1-6, wherein the plasminogen comprises an amino acid sequence having at least 75%, 80%, 85%, 90%, 95%, 96%, 97%, 98%, or 99% sequence identity with the active fragment of plasminogen represented by SEQ ID NO: 14, and has the proteolytic activity of plasminogen.

In some embodiments, the plasminogen is a conservative substitution variant of plasminogen of SEQ ID NO: 2.

9. The method, use, or medicament according to any one of items 1-6, wherein the plasminogen is selected from Glu-plasminogen, Lys-plasminogen, small plasminogen, microplasminogen, delta-plasminogen, or a variant thereof retaining plasminogen activity.

10. The method, use, or medicament according to any one of items 1-6, wherein the plasminogen is natural or synthetic human plasminogen, or a variant or fragment thereof still retaining plasminogen activity.

11. The method, use, or medicament according to any one of items 1-10, wherein the compound is used in combination with one or more other therapeutic methods or medicaments.

12. The method, use, or medicament according to item 11, wherein the other therapeutic methods include surgical treatments, cell therapies (including stem cell therapies), and physical therapies (e.g., physical supportive therapy, e.g., medical device assisted therapy, e.g., human machine interface technology).

13. The method, use, or medicament according to item 11, wherein the other medicaments include hormones, immunosuppressant, neurotrophic medicament, antibiotic, and antiviral medicament.

14. The method, use, or medicament according to any one of items 1-13, wherein the compound is administered by any one or more means or routes selected from the group consisting of: nasal inhalation, aerosol inhalation, nasal drop, eye drops, ear drops, intravenous administration, intraperitoneal administration, subcutaneous administration, intracranial administration, intrathecal administration, intraarterial administration and intramuscular administration.

In any of the above embodiments of the application, the plasminogen may have at least 75%, 80%, 85%, 90%, 95%, 96%, 97%, 98% or 99% sequence identity with SEQ ID NO: 2, 6, 8, 10 or 12, and still have plasminogen activity, e.g., proteolytic activity, lysine binding activity, or both proteolytic activity and lysine binding activity. In some embodiments, the plasminogen is a protein with addition, deletion and/or substitution of 1-100, 1-90, 1-80, 1-70, 1-60, 1-50, 1-45, 1-40, 1-35, 1-30, 1-25, 1-20, 1-15, 1-10, 1-5, 1-4, 1 -3, 1-2, or 1 amino acid based on SEQ ID NO: 2, 6, 8, 10 or 12, and still has plasminogen activity such as proteolytic activity, lysine binding activity, or both proteolytic activity and lysine binding activity.

In some embodiments, the plasminogen is a protein comprising an active fragment of plasminogen and still having plasminogen activity. In some embodiments, the plasminogen is selected from the group consisting of: Glu-plasminogen, Lys-plasminogen, mini-plasminogen, micro-plasminogen, delta-plasminogen, or variants thereof retaining plasminogen activity. In some embodiments, the plasminogen is natural or synthetic human plasminogen, or a variant or fragment thereof still retaining plasminogen activity. In some embodiments, the plasminogen is human plasminogen ortholog from a primate or rodent, or a variant or fragment thereof still retaining plasminogen activity. In some embodiments, the amino acid sequence of the plasminogen is represented by SEQ ID NO: 2, 6, 8, 10 or 12. In some embodiments, the plasminogen is a human natural plasminogen.

In some embodiments, the subject is a human. In some embodiments, the subject is deficient or lacking in plasminogen. In some embodiments, the lack or deficiency is congenital, secondary and/or local.

In some embodiments, the pharmaceutical composition comprises a pharmaceutically acceptable carrier and plasminogen for use in the above methods. In some embodiments, the kit may be a prophylactic or therapeutic kit, comprising: (i) plasminogen for use in the above methods, and (ii) means for delivering the plasminogen to the subject. In some embodiments, the means is a syringe or a vial. In some embodiments, the kit further comprises a label or instructions for administering the plasminogen to the subject to perform any of the above methods.

In some embodiments, the product comprises: a container comprising a label; and further comprises (i) plasminogen for use in the above method or a pharmaceutical composition comprising plasminogen, wherein the label instructs the administration of the plasminogen or composition to the subject to perform any of the above methods.

In some embodiments, the kit or product further comprises one or more additional means or containers containing other medicaments.

In some embodiments of the above methods, the plasminogen is administered by systemic or topical administration for therapy, preferably by intravenous, intramuscular, or subcutaneous administration of plasminogen. In some embodiments of the above methods, the plasminogen is administered in combination with a suitable polypeptide carrier or a stabilizer. In some embodiments of the above methods, the plasminogen is administered per day at the amount of 0.0001-2000 mg/kg, 0.001-800 mg/kg, 0.01-600 mg/kg, 0.1-400 mg/kg, 1-200 mg/kg, 1-100 mg/kg, or 10-100mg/kg (by per kilogram of body weight); or 0.0001-2000 mg/cm$^2$, 0.001-800 mg/cm$^2$, 0.01-600 mg/cm$^2$, 0.1-400 mg/cm$^2$, 1-200 mg/cm$^2$, 1-100 mg/cm$^2$, or 10-100 mg/cm$^2$ (by per square centimeter of body surface area), preferably repeating at least once, and preferably administering at least daily.

The present application explicitly encompasses all the combinations of the technical features belonging to the embodiments of the present application, and these combined technical solutions have been explicitly disclosed in this application, just as the separately and explicitly disclosed above technical solutions. In addition, the present application also explicitly encompasses the combinations of each embodiment and its elements, and the combined technical solutions are explicitly disclosed herein.

**BRIEF DESCRIPTION OF THE DRAWINGS**

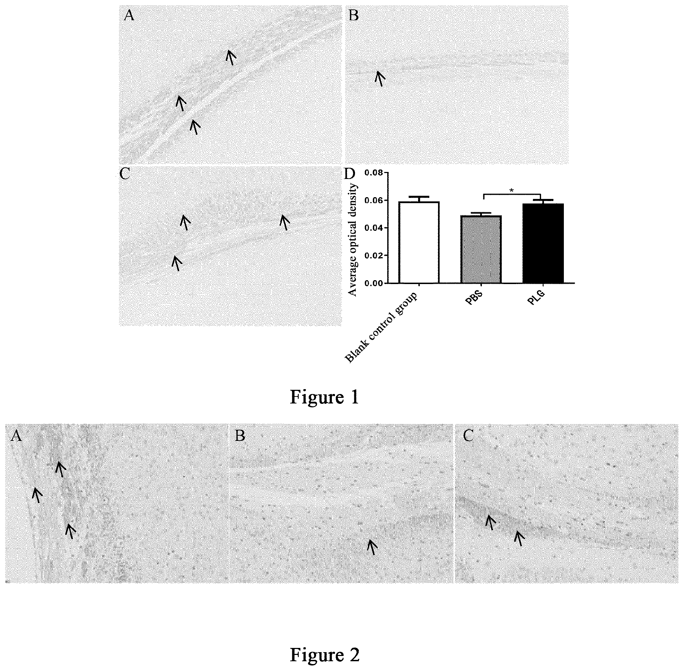

Figure 1A-D show the results of LFB staining of corpus callosum after administrating plasminogen to the model mice of bis(cyclohexanone) oxaldihydrazone induced demyelination for 14 days. A is the blank control group, B is the control group in which the mice are injected with the vehicle PBS *(hereinafter referred to as* vehicle PBS control group, or vehicle group), C is the group in which the mice are injected with plasminogen *(hereinafter referred to as* plasminogen group), and D is the result of quantitative analysis. The results show that the morphology of corpus callosum myelin sheath is basically normal in the blank control group, and the positive staining of corpus callosum myelin sheath (marked by arrows) is significantly more in the plasminogen group than in the vehicle group, and the statistical difference is significant (* means $P<0.05$). It indicates that plasminogen can reduce corpus callosum demyelination and promote the repair of myelin injury in bis(cyclohexanone) oxaldihydrazone induced demyelination model mice.

Figure 2A-C show the representative figures of immunostaining of proteolipid protein (PLP) in corpus callosum after administrating plasminogen to the model mice of bis(cyclohexanone) oxaldihydrazone induced demyelination for 3 days. A is the blank control group, B is the vehicle group, and C is the plasminogen group. The results show that the expression of PLP in the corpus callosum of the plasminogen group (marked by arrows) is significantly more than that of the vehicle group; Comparing to the vehicle group, the expression in the plasminogen group is more similar to that of mice in the blank control group. It indicates that plasminogen promotes the expression of PLP in corpus callosum and promotes the regeneration of myelin sheath in bis(cyclohexanone) oxaldihydrazone induced demyelination model mice.

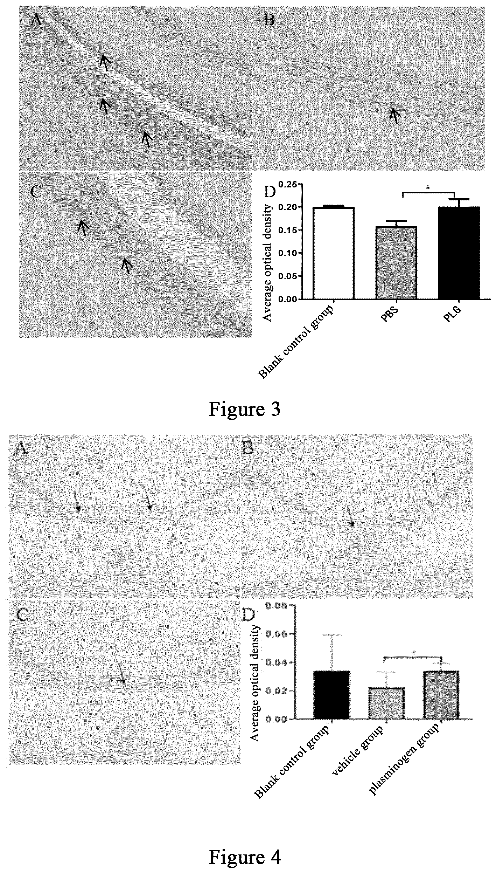

Figure 3A-D show the result of immunostaining of brain neurofilament protein (NFP) after administrating of plasminogen to the model mice of bis(cyclohexanone) oxaldihydrazone induced demyelination for 14 days. A is the blank control group, B is the vehicle group, C is the plasminogen group, and D is the result of quantitative analysis. The results show that the expression of NFP (marked by arrows) in the corpus callosum of mice in the plasminogen group is significantly more than that in the vehicle group, and the statistical difference is significant (*$P<0.05$), and compared to that in the vehicle PBS control group, the expression of NFP in the corpus callosum of mice in plasminogen group is more similar to that in the blank control group. This indicates that plasminogen can promote the expression of NFP, thereby promoting nerve fiber regeneration.

Figure 4 shows the result of immunohistochemical staining of NFP in corpus callosum after administrating of plasminogen to the model mice of multiple sclerosis for 35 days. A is the blank control group, B is the vehicle group, C is the plasminogen group, and D is the quantitative analysis result of mean optical density. The results show that the blank control group expresses a certain level of NFP in the corpus callosum (marked by arrows), the expression of NFP in the corpus callosum of the mice in the vehicle group is significantly reduced, the level of NFP expression in the corpus callosum of mice in the plasminogen group is significantly higher than that in the mice in the vehichle group, and the statistical difference is significant (* indicates $P<0.05$). This result suggests that plasminogen can promote the expression of NFP in the corpus callosum of multiple sclerosis model mice.

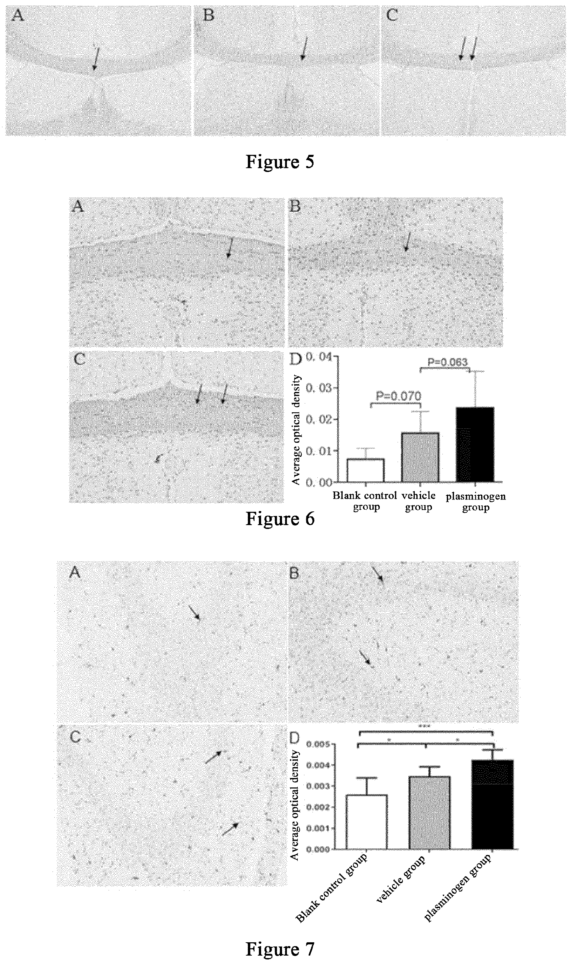

Figure 5A-C show the result of immunohistochemical staining results of PLP in corpus callosum after administrating of plasminogen to the model mice of multiple sclerosis for 35 days. A is the blank control group, B is the vehicle group, and C is the plasminogen group. The results show that the blank control group expresses a certain level of PLP in the corpus callosum (marked by arrows), the expression of PLP in the corpus callosum of the mice in the vehicle group is significantly reduced, and the expression level of PLP in the corpus callosum of the mice in the administration group (plasminogen group) is significantly higher than that in the vehicle group. This result suggests that plasminogen can promote the expression of PLP in the corpus callosum of multiple sclerosis model mice.

Figure 6A-D show the result of immunohistochemical staining results of MBP in corpus callosum after administrating plasminogen to the model mice of multiple sclerosis for 35 days. A is the blank control group, B is the vehicle group, C is the plasminogen group, and D is the quantitative analysis result of mean optical density. The results show that the blank control group expresses a certain level of MBP in the corpus callosum (marked by arrows), and the MBP in the corpus callosum of the mice in the plasminogen group is significantly more than that of the mice in the vehicle group, and the statistical difference is close to significant (P=0.063). This result suggests that plasminogen can promote the increase of MBP level in the corpus callosum of multiple sclerosis model mice.

Figure 7A-D show the result of immunohistochemical staining results of Iba-1 in hippocampi after administrating plasminogen to the model mice of multiple sclerosis for 35 days. A is the blank control group, B is the vehicle group, C is the plasminogen group, and D is the quantitative analysis result of mean optical density. The results show that there is a certain amount of microglia in the hippocampi of mice in the blank control group (marked by arrows), and the amount of microglia in the hippocampi of mice in the vehicle group is significantly increased, and the number of microglia in the hippocampi of mice in the plasminogen group is significantly more than that in the vehicle group, and the statistical differences are significant (* indicates P<0.05, *** indicates P<0.001). It is suggested that plasminogen can promote repair of hippocampal injury inflammation in multiple sclerosis model mice.

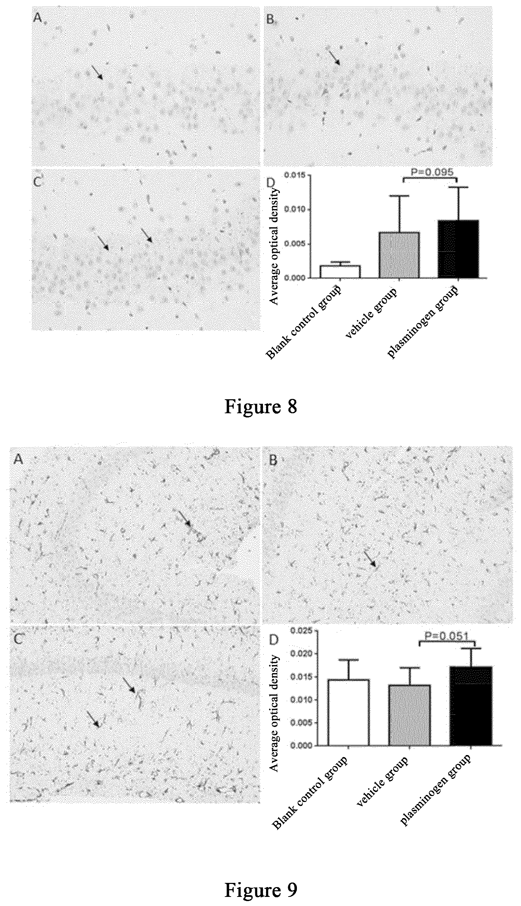

Figure 8A-D show the result of immunohistochemical staining results of BDNF in hippocampi after administrating plasminogen to the model mice of multiple sclerosis for 35 days. A is the blank control group, B is the vehicle group, C is the plasminogen group, and D is the quantitative analysis result of mean optical density. The results show that the hippocampi of mice in the blank control group have a certain level of BDNF (marked by arrows), the hippocampi of mice in the vehicle group have an increased level of BDNF, and the level of BDNF in the hippocampi of mice in the plasminogen group is significantly higher than that in the vehicle group, and the statistical difference is close to significant (P=0.095). It suggests that plasminogen can promote the increase of BDNF level in hippocampi of multiple sclerosis model mice.

Figure 9A-D show the result of immunohistochemical staining results of GFAP in hippocampi after administrating plasminogen to the model mice of multiple sclerosis for 35 days. A is the blank control group, B is the vehicle group, C is the plasminogen group, and D is the quantitative analysis result of mean optical density. The results show that the hippocampi of mice in the blank control group expresses a certain level of GFAP (marked by arrows), the hippocampi of mice in the vehicle group expressed reduced GFAP, and the hippocampi of mice in the plasminogen group expresses significantly more GFAP than that in the vehicle group, and the statistical difference is close to significant (P=0.051). It suggests that plasminogen can promote the increase of GFAP expression in hippocampi and astrocyte activity of multiple sclerosis model mice.

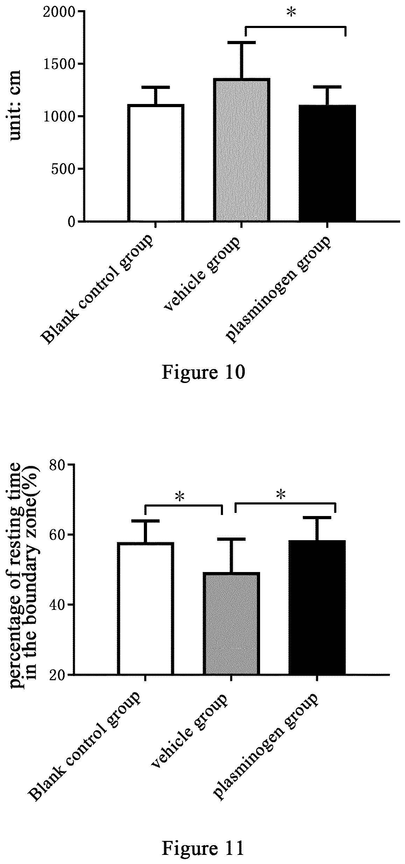

Figure 10 shows the statistical results of the total movement distance in the open field experiment after administrating plasminogen to the model mice of multiple sclerosis for 28 days. The results show that the mice in the blank control group have a certain total movement distance, and the mice in the vehicle group have a significantly increased total movement distance. The total movement distance of the mice in the plasminogen group is significantly less than that in the vehicle group, and the statistical difference is significant (* indicates P<0.05), and it's similar to that in the blank control group. It indicates that plasminogen can alleviate depressive behavior in multiple sclerosis model mice.

Figure 11 shows the percentage of resting time in the boundary zone = resting time in the boundary zone/total observation time. The results show that the mice in the blank control group have a certain percentage of resting time in the boundary zone, which is about 57.8%, and the percentage of resting time in the boundary zone of the mice in the vehicle group is significantly decreased, which is about 49.3%. The percentage of resting time in the boundary zone of the mice in the plasminogen group is about 58.4%, which is significantly greater than that in the vehicle group, and the statistical difference is significant (* indicates P<0.05), and and it's similar to that in the blank control group. It indicates that plasminogen can alleviate the depressive behavior of multiple sclerosis model mice to some extent.

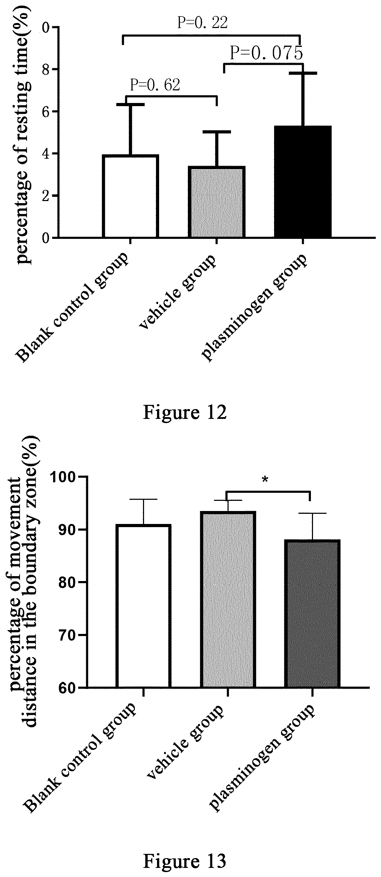

Figure 12 shows the statistical results of the percentage of resting time in the contact range of unfamiliar mouse 2 in the three-chamber test after administrating plasminogen to the model mice of multiple sclerosis for 34 days. Percentage of resting time = resting time/total observation time. The results of the second phase of the three-chamber sociability test show that the mice in the blank control group has a certain percentage of resting time in the contact range of unfamiliar mouse 2, which is about 13.7%; that in the vehicle group is significantly decreased, which is about 10.6%; that in the plasminogen group is about 16.1%, which is significantly greater than that in the vehicle group, and the statistical difference is close to significant (P=0.075). It indicates that plasminogen can improve the social memory in multiple sclerosis model mice.

Figure 13 shows the statistical results of the percentage of movement distance in the boundary zone in the open field experiment after administrating plasminogen to the model rats of the multiple sclerosis for 6 days. The movement distance in the boundary zone is the length of the motion trail in the boundary zone during the test time of the open field experiment. The results show that the blank control group has a certain percentage of movement distance in the boundary zone, which is about 91.1%; that of the vehicle group is significantly increased, which is about 93.6%; that of the plasminogen group is about 88.1%, which is significantly less than that of the vehicle group, and the statistical difference is significant (* indicates P<0.05). It indicates that plasminogen can alleviate the depressive behavior of multiple sclerosis model mice.

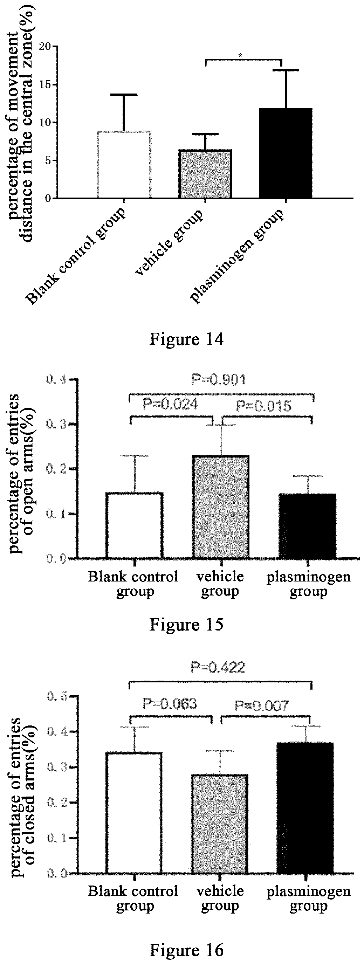

Figure 14 shows the statistical results of the percentage of movement distance in the central zone in the open field experiment after administrating plasminogen to the model rats of multiple sclerosis for 6 days. The results show that the blank control group has a certain percentage of central zone distance, which is about 8.9%; that of the vehicle group is significantly decreased, which is about 6.4%; that of the plasminogen group is about 11.9%, which is significantly greater than that of the vehicle group, and the statistical difference is significant (* indicates P<0.05). It indicates that plasminogen can alleviate the anxious behavior of multiple sclerosis model rats.

Figure 15 shows the statistical results of the percentage of entries of open arms in the elevated cross-maze test after administrating plasminogen to the model mice of multiple sclerosis for 20 days. The results show that the mice in the blank control group have a certain percentage of entries of open arms, which is about 14.9%; the percentage of entries of open arms in the vehicle group is significantly increased, which is about 23.0%; the percentage of entries of open arms in the plasminogen group is about 14.5%, which is significantly less than that in the vehicle group, and the statistical difference is significant (P=0.015), and it's similar to that of the blank control group. It indicates that plasminogen can alleviate the anxious behavior of multiple sclerosis model mice to some extent.

Figure 16 shows the statistical results of the percent of entries of closed arms in the elevated cross-maze test after administrating plasminogen to the model mice of multiple sclerosis for 20 days. The results show that the mice in the blank control group have a certain percent of entries of closed arms, which is about 34.4%; the percent of entries of closed arms in the vehicle group is significantly decreased, which is about 28.1%; the percent of entries of closed arms of the mice in the plasminogen group is about 37.1%, which is significantly greater than that in the vehicle group, and the statistical difference is significant (P=0.007), and it's similar to that in the blank control group. It indicates that plasminogen can alleviate the anxious and depressive behavior of multiple sclerosis model mice to some extent.

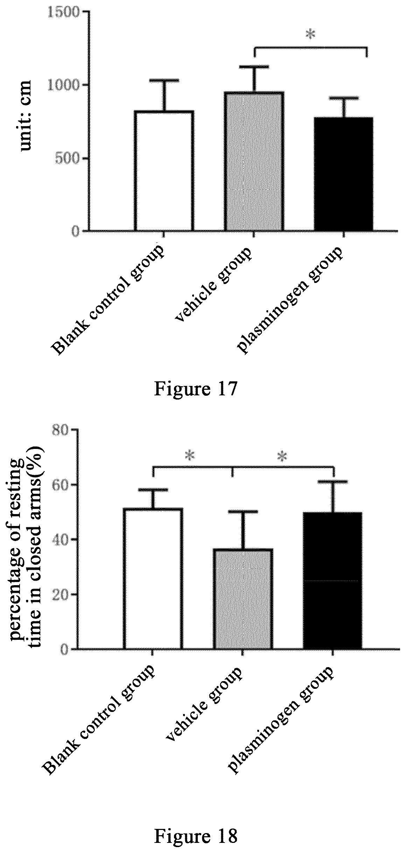

Figure 17 shows the statistical results of the movement distance in closed arms in the elevated cross-maze test after administrating plasminogen to the model mice of multiple sclerosis for 27 days. The total movement distance in closed arms is the total movement distance of mice in closed arms during the test time of the elevated cross-maze test. The results show that the mice in the blank control group have a certain total movement distance in closed arms, and the total movement distance of the mice in the vehicle group in closed arms is significantly increased, and the total distance movement of the mice in the plasminogen group in closed arms is significantly less than that in the vehicle group, and the statistical difference is significant (* indicates P<0.05) and it's similar to that in the blank control group. It indicates that plasminogen can alleviate the anxious and depressive behavior of multiple sclerosis model mice to some extent.

Figure 18 shows the statistical results of the percentage of resting time in closed arms in the elevated cross-maze test after administrating plasminogen to the model mice of multiple sclerosis for 27 days. The results show that the mice in the blank control group have a certain percentage of resting time in closed arms, which is about 51.8%; the percentage of resting time in closed arms in the vehicle group is significantly decreased, which is about 36.8%; the percentage of resting time in closed arms in the plasminogen group is about 50.1%, which is significantly greater than that in the vehicle group, and the statistical difference is significant (* indicates P<0.05) and it's similar to that in the blank control group. It indicates that plasminogen can alleviate the anxious and depressive behavior of multiple sclerosis model mice to some extent.

## DETAIL DESCRIPTION OF THE APPLICATION

Fibrinolytic system is a system consisting of a series of chemical substances involved in the process of fibrinolysis, mainly including plasminogen, plasmin, plasminogen activator, and fibrinolysis inhibitor. Plasminogen activators include tissue-type plasminogen activator (t-PA) and urokinase-type plasminogen activator (u-PA). t-PA is a serine protease that is synthesized by vascular endothelial cells. t-PA activates plasminogen, which is mainly carried out on fibrin; urokinase-type plasminogen activator (u-PA) is produced by renal tubular epithelial cells and vascular endothelial cells, and may directly activate plasminogen without the need for fibrin as a cofactor. Plasminogen (PLG) is synthesized by liver. When blood coagulates, a large amount of PLG is adsorbed on the fibrin network, and under the action of t-PA or u-PA it is activated into plasmin to promote fibrinolysis. Plasmin (PL) is a serine protease whose functions are as follows: degrading fibrin and fibrinogen; hydrolyzing various coagulation factors V, VIII, X, VII, XI, and II, etc.; converting plasminogen into plasmin; hydrolyzing complement, etc. Fibrinolysis inhibitors: including plasminogen activator inhibitor (PAI) and α2 antiplasmin (α2-AP). PAI mainly has two forms, PAI-1 and PAI-2, which may specifically bind to t-PA in a ratio of 1:1, thereby inactivating it and activating PLG at the same time. α2-AP is synthesized by liver, and binds to PL in a ratio of 1:1 to form a complex to inhibit the activity of PL; FXIII makes α2-AP covalently bound to fibrin, reducing the sensitivity of fibrin to PL. Substances that inhibit the activity of the fibrinolytic system in vivo: PAI-1, complement C1 inhibitor; α2 antiplasmin; α2 macroglobulin.

The term "component of plasminogen activation pathway" according to the present application encompasses:

1. plasminogen, Lys-plasminogen, Glu-plasminogen, micro-plasminogen, delta-plasminogen; variants or analogs thereof;
2. plasmin and a variant or analog thereof; and
3. plasminogen activators, such as tPA and uPA, and tPA or uPA variants and analogs comprising one or more domains of tPA or uPA, such as one or more kringle domains and proteolytic domains.

"Variants" of the above plasminogen, plasmin, tPA and uPA include all naturally occurring human genetic variants as well as other mammalian forms of these proteins, as well as a protein obtained by addition, deletion and/or substitution of such as 1-100, 1-90, 1-80, 1-70, 1-60, 1-50, 1-45, 1-40, 1-35, 1-30, 1-25, 1-20, 1-15, 1-10, 1-5, 1-4, 1-3, 1-2, or 1 amino acid, and still retaining the activity of plasminogen, plasmin, tPA or uPA. For example, "variants" of plasminogen, plasmin, tPA and uPA include mutational variants of these proteins obtained by substitution of such as 1-100, 1-90, 1-80, 1-70, 1-60, 1-50, 1- 45, 1-40, 1-35, 1-30, 1-25, 1-20, 1-15, 1-10, 1-5, 1-4, 1-3, 1-2, or 1 conservative amino acid.

A "plasminogen variant" of the application encompasses a protein having at least 75%, 80%, 85%, 90%, 95%, 96%, 97%, 98% or 99% sequence identity with SEQ ID NO: 2, 6, 8, 10 or 12, and still retaining plasminogen activity. For example, a "plasminogen variant" according to the present application may be a protein obtained by addition, deletion and/or substitution of 1-100, 1-90, 1-80, 1-70, 1-60, 1-50, 1-45, 1-40, 1-35, 1-30, 1-25, 1-20, 1-15, 1-10, 1-5, 1-4, 1-3, 1-2, or 1 amino acid on the basis of SEQ ID NO: 2, 6, 8, 10 or 12, and still retaining plasminogen activity. Particularly, the plasminogen variants according to the present application include all naturally occurring human genetic variants as well as other mammalian forms of these proteins, as well as mutational variants of these proteins obtained by substitution of such as 1-100, 1-90, 1-80, 1-70, 1-60, 1-50, 1-45, 1-40, 1-35, 1-30, 1-25, 1-20, 1-15, 1-10, 1-5, 1-4, 1-3, 1-2, or 1 conservative amino acid.

The plasminogen according to the present application may be a human plasminogen ortholog from a primate or rodent, or a variant thereof still retaining plasminogen activity, for example, a plasminogen represented by SEQ ID NO: 2, 6, 8, 10 or 12, such as a human natural plasminogen represented by SEQ ID NO: 2.

The "analogs" of the above plasminogen, plasmin, tPA, and uPA include compounds that respectively provide substantially similar effect to plasminogen, plasmin, tPA, or uPA.

The "variants" and "analogs" of above plasminogen, plasmin, tPA and uPA encompass "variants" and "analogs" of plasminogen, plasmin, tPA and uPA comprising one or more domains (e.g., one or more kringle domains and proteolytic domains). For example, "variants" and "analogs" of plasminogen encompass "variants" and "analogs" of plasminogen comprising one or more plasminogen domains (e.g., one or more kringle domains and proteolytic domains), such as mini-plasminogen. "Variants" and "analogs" of plasmin encompass "variants" and "analogs" of plasmin comprising one or more plasmin domains (e.g., one or more kringle domains and proteolytic domains), such as mini-plasmin, and delta-plasmin.

Whether a "variant" or "analog" of the above plasminogen, plasmin, tPA or uPA respectively has the activity of plasminogen, plasmin, tPA or uPA, or whether the "variant" or "analog" provides substantially similar effect to plasminogen, plasmin, tPA or uPA, may be detected by methods known in the art, for example, it is measured by the level of activated plasmin activity based on enzymography, ELISA (enzyme-linked immunosorbent assay), and FACS (fluorescence-activated cell sorting method), for example, it is detected by referring to a method selected from the following documents: Ny, A., Leonardsson, G., Hagglund, A.C, Hagglof, P., Ploplis, V.A., Carmeliet, P. and Ny, T. (1999). Ovulation inplasminogen-deficient mice. Endocrinology 140, 5030-5035; Silverstein RL, Leung LL, Harpel PC, Nachman RL (November 1984). "Complex formation of platelet thrombospondin with plasminogen. Modulation of activation by tissue activator". J. Clin. Invest.74(5):1625-33; Gravanis I, Tsirka SE (February 2008). "Tissue-type plasminogen activator as a therapeutic target in stroke". Expert Opinion on Therapeutic Targets. 12(2):159-70; Geiger M, Huber K, Wojta J, Stingl L, Espana F, Griffin JH, Binder BR (Aug 1989). "Complex formation between urokinase and plasma protein C inhibitor in vitro and in vivo". Blood.74(2):722-8.

In some embodiments of the present application, the "component of plasminogen activation pathway" according to the present application is a plasminogen selected from the group consisting of: Glu-plasminogen, Lys-plasminogen, mini-plasminogen, micro-plasminogen, delta-plasminogen, or variants thereof retaining plasminogen activity. In some embodiments, the plasminogen is natural or synthetic human plasminogen, or a conservative mutant variant or fragment thereof still retaining plasminogen activity. In some embodiments, the plasminogen is a human plasminogen ortholog from a primate or rodent or a conservative mutant variant or fragment thereof still retaining plasminogen activity. In some embodiments, the amino acid sequence of the plasminogen is represented by SEQ ID NO: 2, 6, 8, 10 or 12. In some embodiments, the plasminogen is a human natural plasminogen. In some embodiments, the plasminogen is a human natural plasminogen represented by SEQ ID NO: 2.

"A compound capable of directly activating plasminogen, or indirectly activating plasminogen by activating an upstream component of plasminogen activation pathway", refers to any compound capable of directly activating plasminogen, or indirectly activating plasminogen by activating an upstream component of plasminogen activation pathway, such as tPA, uPA, streptokinase, saruplase, alteplase, reteplase, tenecteplase, anistreplase, monteplase, lanoteplase, pamiteplase, staphylokinase.

The "antagonist of a fibrinolysis inhibitor" according to the present application is a compound that antagonizes, weakens, blocks, or prevents the action of a fibrinolysis inhibitor. Such fibrinolysis inhibitors are e.g., PAI-1, complement C1 inhibitor, $\alpha$2 antiplasmin, and $\alpha$2 macroglobulin. Such an antagonist is: e.g., an antibody of PAI-1, complement C1 inhibitor, $\alpha$2 antiplasmin, or $\alpha$2 macroglobulin; or an antisense RNA or small RNA blocking or downregulating the expression of such as PAI-1, complement C1 inhibitor, $\alpha$2 antiplasmin or $\alpha$2 macroglobulin; or a compound occupying the binding site of PAI-1, complement C1 inhibitor, $\alpha$2 antiplasmin, or $\alpha$2 macroglobulin but without the function of PAI-1, complement C1 inhibitor, $\alpha$2 antiplasmin, or $\alpha$2 macroglobulin; or a compound blocking the binding domains and/or active domains of PAI-1, complement C1 inhibitor, $\alpha$2 antiplasmin, or $\alpha$2 macroglobulin.

Plasmin is a key component of the plasminogen activation system (PA system). It is a broad-spectrum protease capable of hydrolyzing several components of the extracellular matrix (ECM), including fibrin, gelatin, fibronectin, laminin, and proteoglycans. In addition, plasmin may activate some metalloproteinase precursors (pro-MMPs) to form active metalloproteinases (MMPs). Therefore, plasmin is considered to be an important upstream regulator of extracellular proteolysis. Plasmin is formed by proteolysis of plasminogen by two physiological PAs: tissue-type plasminogen activator (tPA) or urokinase-type plasminogen activator (uPA). Due to the relatively high levels of plasminogen in plasma and other body fluids, it has traditionally been thought that the regulation of the PA system is mainly achieved through the synthesis and activity levels of PAs. The synthesis of components of PA system is strictly regulated by different factors, such as hormone, growth factor and cytokine. In addition, there are specific physiological inhibitors of plasmin and PAs. The main inhibitor of plasmin is $\alpha$2-antiplasmin. The activity of PAs is inhibited by plasminogen activator inhibitor-1 (PAI-1) of both uPA and tPA, and regulated by plasminogen activator inhibitor-2 (PAI-2) which mainly inhibits uPA. Certain cell surfaces have uPA-specific cell surface receptors (uPARs) with direct hydrolytic activity.

Human plasminogen is a single-chain glycoprotein consisting of 791 amino acids with a molecular weight of approximately 92 kDa. Plasminogen is mainly synthesized in liver, and is abundantly present in the extracellular fluid. The content of plasminogen in plasma is approximately 2 $\mu$M. Plasminogen is thus a huge potential source of proteolytic activity in tissues and body fluids. Plasminogen exists in two molecular forms: glutamate-plasminogen (Glu-plasminogen) and lysine-plasminogen (Lys-plasminogen). The naturally secreted and uncleaved form of plasminogen has an amino-terminal (N-terminal) glutamate, and is therefore referred to as glutamate-plasminogen. However, in the presence of plasmin, glutamate-plasminogen is hydrolyzed at Lys76-Lys77 into lysine-plasminogen. Compared with glutamate-plasminogen, lysine-plasminogen has a higher affinity for fibrin, and may be activated by PAs at a higher rate. The Arg560-Val561 peptide bond of these two forms of plasminogen may be cleaved by either uPA or tPA, resulting in the formation of a two-chain protease plasmin linked by disulfide. The amino-terminal part of plasminogen comprises five homologous tri-cycles, i.e., so-called kringles, and the carboxy-terminal part comprises the protease domain. Some kringles comprise lysine-binding sites that mediate the specific interaction of plasminogen with fibrin and its inhibitor $\alpha$2-AP. A recently found plasminogen is a 38 kDa fragment, including kringles1-4, and it is a potent inhibitor of angiogenesis. This fragment is named as angiostatin, and is produced by the hydrolysis of plasminogen by several proteases.

The main substrate of plasmin is fibrin, and the dissolution of fibrin is the key to preventing pathological thrombosis. Plasmin also has substrate specificity for several components of the ECM, including laminin, fibronectin, proteoglycans, and gelatin, indicating that plasmin also plays an important role in ECM remodeling. Indirectly, plasmin may also degrade other components of the ECM, including MMP-1, MMP-2, MMP-3 and MMP-9, by converting certain protease precursors into active proteases. Therefore, it has been proposed that plasmin may be an important upstream regulator of extracellular proteolysis. In addition, plasmin has the ability to activate certain latent forms of growth factors. In vitro, plasmin also hydrolyzes components of the complement system, and releases chemotactic complement fragments.

"Plasmin" is a very important enzyme present in the blood that hydrolyzes fibrin clots into fibrin degradation products and D-dimers.

"Plasminogen" is the zymogen form of plasmin. According to the sequence in swiss prot, it consists of 810 amino acids calculating by the natural human plasminogen amino acid sequence (SEQ ID NO: 4) containing the signal peptide, and the molecular weight is about 90kD, and it is a glycoprotein mainly synthesized in the liver and capable of circulating in the blood, the cDNA sequence encoding this amino acid sequence is represented by SEQ ID NO: 3. Full-length plasminogen contains seven domains: a C-terminal serine protease domain, an N-terminal Pan Apple (PAp) domain, and five Kringle domains (Kringle1-5). Referring to the sequence in swiss prot, its signal peptide comprises residues Met1-Gly19, PAp comprises residues Glu20-Val98, Kringle1 comprises residues Cys103-Cys181, Kringle2 comprises residues Glu184-Cys262, Kringle3 comprises residues Cys275-Cys352, Kringle4 comprises residues Cys377-Cys454, and Kringle5 comprises residues Cys481-Cys560. According to NCBI data, the serine protease domain comprises residues Val581-Arg804.

Glu-plasminogen is a human natural full-length plasminogen, consisting of 791 amino acids (without a signal peptide of 19 amino acids); the cDNA sequence encoding this amino acid sequence is represented by SEQ ID NO: 1, and the amino acid sequence is represented by SEQ ID NO: 2. In vivo, there is also a Lys-plasminogen produced by the hydrolysis of the peptide bond between amino acids 76 and 77 of Glu-plasminogen, as represented by SEQ ID NO: 6; and the cDNA sequence encoding this amino acid sequence is represented by SEQ ID NO: 5. Delta-plasminogen (δ-plasminogen) is a fragment of full-length plasminogen that lacks the Kringle2-Kringle5 structure, and only contains Kringle1 and a serine protease domain (also known as a protease domain (PD)). The amino acid sequence of delta-plasminogen (SEQ ID NO: 8) is reported in a literature, and the cDNA sequence encoding this amino acid sequence is represented by SEQ ID NO: 7. Mini-plasminogen consists of Kringle5 and a serine protease domain, and it is reported that it comprises residues Val443-Asn791 (with the Glu residue of the Glu-plasminogen sequence without the signal peptide as the starting amino acid), the amino acid sequence of the mini-plasminogen is represented by SEQ ID NO: 10, and the cDNA sequence encoding this amino acid sequence is represented by SEQ ID NO: 9. While micro-plasminogen comprises only a serine protease domain, and it is reported that its amino acid sequence comprises residues Ala543-Asn791 (with the Glu residue of the Glu-plasminogen sequence without the signal peptide as the starting amino acid); additionally, it is disclosed in patent document CN102154253A that its sequence comprises residues Lys531-Asn791 (with the Glu residue of the Glu-plasminogen sequence without the signal peptide as the starting amino acid); in the present patent application, the sequence of micro-plasminogen refers to the patent document CN102154253A, the amino acid sequence is represented by SEQ ID NO: 12, and the cDNA sequence encoding this amino acid sequence is represented by SEQ ID NO: 11.

The structure of the full-length plasminogen is also described in the article by Aisina et al. (Aisina RB, Mukhametova L I. Structure and function of plasminogen/plasmin system [J]. Russian Journal of Bioorganic Chemistry, 2014, 40(6):590-605). In this article, Aisina et al. describe that plasminogen comprises Kringle 1, 2, 3, 4, 5 domains and a serine protease domain (also called protease domain (PD)), wherein Kringles are responsible for binding of plasminogen to low or high molecular weight ligand (i.e. lysine binding activity), so that plasminogen transforms into a more open conformation that is more readily activated; the protease domain (PD) is residues Val562-Asn791; the Arg561-Val562 activating bond of plasminogen is specifically cleaved by tPA and uPA, thereby allowing plasminogen to change into plasmin, thus the protease domain (PD) is the region that confers the proteolytic activity of plasminogen.

In the present application, "plasmin" and "fibrinolytic enzyme" may be used interchangeably with the same meaning; "plasminogen" and "fibrinolytic zymogen" may be used interchangeably with the same meaning.

In the present application, "lack" of plasminogen or plasminogen activity means that the content of plasminogen in a subject is lower than that of a normal person, and is sufficiently low to affect the normal physiological function of the subject; "deficiency" of plasminogen or plasminogen activity means that the content of plasminogen in a subject is significantly lower than that of a normal person, and even the activity or expression is extremely low, and the normal physiological function may only be maintained by external supply of plasminogen.

Those skilled in the art may understand that, all technical solutions of plasminogen according to the present application are applicable to plasmin, thus the technical solutions described in the present application encompass plasminogen and plasmin. During circulation, plasminogen is present in a closed, inactive conformation, but when bound to a thrombus or cell surface, it is converted into active plasmin with an open conformation after being mediated by plasminogen activator (PA). Active plasmin may further hydrolyze the fibrin clot into degradation products of fibrin and D-dimers, thereby dissolving the thrombus. The PAp domain of plasminogen comprises an important determinant for maintaining plasminogen in an inactive closed conformation, while the KR domain may bind to a lysine residue present on a receptor and substrate. A variety of enzymes are known to act as plasminogen activators, including: tissue plasminogen activator (tPA), urokinase plasminogen activator (uPA), kallikrein, and coagulation factor XII (Hageman factor) etc.

An "active fragment of plasminogen" refers to a fragment having the activity of binding to a lysine in the target sequence of a substrate (lysine-binding activity), or exerting the activity of a proteolytic function (proteolytic activity), or having a combination of proteolytic activity and lysine-binding activity. The technical solutions related to plasminogen according to the present application encompass the technical solutions of replacing plasminogen with an active fragment of plasminogen. In some embodiments, the active fragment of plasminogen according to the present application comprises or consists of a serine protease domain of plasminogen, preferably the active fragment of plasminogen according to the present application comprises or consists of SEQ ID NO: 14, or an amino acid sequence having at least 80%, 90%, 95%, 96%, 97%, 98%, 99% identity with SEQ ID NO: 14. In some embodiments, the active fragment of plasminogen according to the present application comprises or consists of one or more regions selected from the group consisting of: Kringle 1, Kringle 2, Kringle 3, Kringle 4, and Kringle 5. In some embodiments, the plasminogen according to the present application comprises a protein comprising the active fragment of plasminogen described above.

At present, the methods for measuring plasminogen and its activity in blood comprise: detection of tissue plasminogen activator activity (t-PAA), detection of plasma tissue plasminogen activator antigen (t-PAAg), detection of plasma tissue plasminogen activity (plgA), detection of plasma tissue plasminogen antigen (plgAg), detection of the activity of plasma tissue plasminogen activator inhibitor, detection of the antigen of plasma tissue plasminogen activator inhibitor, and detection of plasma plasmin-antiplasmin complex (PAP); wherein the most commonly used detection method is the chromogenic substrate method: adding streptokinase (SK) and a chromogenic substrate to the plasma to be detected, the PLG in the plasma to be detected is converted into PLM under the action of SK, and PLM acts on the chromogenic substrate; subsequently, the detection by spectrophotometer indicates that the increase in absorbance is proportional to plasminogen activity. In addition, the plasminogen activity in blood may also be detected by immunochemical method, gel electrophoresis, immunoturbidimetry, and radioimmunoassay.

"Ortholog or orthologs" refer to homologs between different species, including both protein homologs and DNA homologs, also known as orthologs and vertical homologs; particularly it refers to proteins or genes evolved from the same ancestral gene in different species. The plasminogen according to the present application includes human natural plasminogen, and also includes plasminogen ortholog or orthologs derived from different species and having plasminogen activity.

A "conservative substitution variant" refers to a variant in which a given amino acid residue is altered without changing the overall conformation and function of the protein or enzyme, including but not limited to those variants in which the amino acid(s) in the amino acid sequence of the parent protein are replaced by amino acid(s) with similar properties (e.g., acidic, basic, hydrophobic, etc.). Amino acids with similar properties are well known in the art. For example, arginine, histidine and lysine are hydrophilic basic amino acids and are interchangeable. Similarly, isoleucine is a hydrophobic amino acid, and may be replaced by leucine, methionine or valine. Therefore, the similarity of two proteins or amino acid sequences with similar functions may differ; for example, 70% to 99% similarity (identity) based on the MEGALIGN algorithm. "Conservative substitution variants" also include polypeptides or enzymes having not less than 60%, preferably not less than 75%, more preferably not less than 85%, or even most preferably not less than 90% amino acid identity determined by BLAST or FASTA algorithm, and having the same or substantially similar properties or functions as the natural or parent protein or enzyme.

"Isolated" plasminogen refers to a plasminogen protein isolated and/or recovered from its natural environment. In some embodiments, the plasminogen will be purified: (1) to more than 90%, more than 95%, or more than 98% purity (by weight), as determined by Lowry's method, e.g., more than 99% (by weight), (2) to a degree sufficient to obtain at least 15 residues of the N-terminal or internal amino acid sequence by using a spinning cup sequence analyzer, or (3) to homogeneity as determined by using Coomassie blue or silver staining through sodium dodecyl sulfate-polyacrylamide gel electrophoresis (SDS-PAGE) under reducing or non-reducing conditions. Isolated plasminogen also includes plasminogen prepared from recombinant cells by bioengineering techniques and isolated by at least one purification step.

The terms "polypeptide", "peptide" and "protein" are used interchangeably herein to refer to a polymeric form of amino acids of any length, which may include genetically encoded and non-genetically encoded amino acids, chemically or biochemically modified or derivatized amino acids, and polypeptides with modified peptide backbones. The terms include fusion proteins including, but not limited to, fusion proteins with heterologous amino acid sequences, fusions with heterologous and homologous leader sequences (with or without N-terminal methionine residues); and the like.

"Percent (%) of amino acid sequence identity" with respect to a reference polypeptide sequence is defined as, after introducing gaps as necessary to achieve maximum percent sequence identity, and no conservative substitutions are considered as part of the sequence identity, the percentage of amino acid residues in a candidate sequence that are identical to the amino acid residues in a reference polypeptide sequence. Alignment for purposes of determining percent amino acid sequence identity may be accomplished in a variety of ways within the technical scope in the art, e.g., by publicly available computer software, such as BLAST, BLAST-2, ALIGN or Megalign (DNASTAR) software. Those skilled in the art may determine the appropriate parameters for aligning sequences, including any algorithms needed to achieve maximal alignment over the full length of the sequences to be compared. However, for the purpose of the present application, the values of percent amino acid sequence identity are generated by using the computer program ALIGN-2 for sequence comparison.

Where ALIGN-2 is used to compare amino acid sequences, the percentage (%) of amino acid sequence identity of a given amino acid sequence A relative to a given amino acid sequence B (or may be expressed as a given amino acid sequence A having a certain percentage (%) of amino acid sequence identity relative to, with or with respective to a given amino acid sequence B) is calculated as follows:

## Fraction X/Y times 100;

wherein X is the number of amino acid residues scored as identical matches during the alignment of sequences A and B by the sequence alignment program ALIGN-2, and wherein Y is the total number of amino acid residues in sequence B. It should be appreciated that, where the length of amino acid sequence A is not equal to that of amino acid sequence B, the percentage (%) of amino acid sequence identity of A with respect to B will not equal to the percentage (%) of amino acid sequence identity of B with respect to A. Unless expressly stated otherwise, all the values of percentage (%) of amino acid sequence identity used herein are obtained by using the ALIGN-2 computer program as described in the preceding paragraph.

As used herein, the terms "treatment /treating" refer to obtaining a desired pharmacological and/or physiological effect. The effect may be complete or partial prevention of the occurrence or onset of the disease or symptoms thereof, partial or complete alleviation of the disease and/or symptoms thereof, and/or partial or complete cure of the disease and/or symptoms thereof; and includes: (a) preventing the occurrence or onset of the disease in a subject, who may have predisposition of the disease, but is not yet diagnosed as having the disease; (b) inhibiting the disease, i.e., blocking its development; and (c) alleviating the disease and/or symptoms thereof, i.e., causing regression or elimination of the disease and/or symptoms thereof.

The terms "individual", "subject" and "patient" are used interchangeably herein to refer to mammals including, but not limited to, murine (rat, mouse), non-human primate, human, canine, feline, hoofed animals (e.g., horses, cattle, sheep, pigs, goats), etc.

A "therapeutically effective amount" or "effective amount" refers to an amount of a component of plasminogen activation pathway or a related compound thereof (e.g. plasminogen) sufficient to prevent and/or treat a disease when administered to a mammal or other subject for treating the disease. A "therapeutically effective amount" will vary depending on the component of the plasminogen activation pathway or a related compound thereof (e.g. plasminogen) in use, the severity of the disease and/or symptoms thereof in the subject to be treated, as well as the age, weight, and the like.

### Preparation of the Plasminogen According to the Present Application

Plasminogen may be isolated from nature, and purified for further therapeutic use, or it may be synthesized by standard chemical peptide synthesis techniques. When the polypeptide is synthesized chemically, the synthesis may be carried out via liquid phase or solid phase. Solid-phase polypeptide synthesis (SPPS) (in which the C-terminal amino acid of the sequence is attached to an insoluble support, followed by the sequential addition of the retaining amino acids in the sequence) is a suitable method for chemical synthesis of plasminogen. Various forms of SPPS, such as Fmoc and Boc, may be used to synthesize plasminogen. Techniques for solid-phase synthesis are described in Barany and Solid-Phase Peptide Synthesis; pp.3-284 in The Peptides: Analysis, Synthesis, Biology. Vol. 2: Special Methods in Peptide Synthesis, Part A., Merrifield, et al. J. Am. Chem. Soc., 85:2149-2156 (1963); Stewart et al., Solid Phase Peptide Synthesis, 2nd ed. Pierce Chem. Co., Rockford, Ill. (1984); and Ganesan A. 2006 Mini Rev. Med Chem. 6:3-10, and Camarero JA et al. 2005, Protein Pept Lett. 12:723-8. Briefly, small insoluble porous beads are treated with functional units on which peptide chains are constructed; after repeated cycles of coupling/deprotection, the attached solid-phase free N-terminal amine is coupled to a single N-protected amino acid unit. This unit is then deprotected to reveal new N-terminal amines that may be attached to other amino acids. The peptide remains immobilized on the solid phase, subsequently it is cleaved off.

Plasminogen according to the present application may be produced by standard recombinant methods. For example, a nucleic acid encoding plasminogen is inserted into an expression vector to be operably linked to regulatory sequences in the expression vector. The regulatory sequences for expression include, but are not limited to, promoters (e.g., naturally associated or heterologous promoters), signal sequences, enhancer elements, and transcription termination sequences. Expression regulation may be a eukaryotic promoter system in a vector capable of transforming or transfecting a eukaryotic host cell (e.g., COS or CHO cell). Once the vector is incorporated into a suitable host, the host is maintained under conditions suitable for high-level expression of the nucleotide sequence and collection and purification of plasminogen.

A suitable expression vector is typically replicated in a host organism as an episome or as an integrated part of the host chromosomal DNA. Typically, an expression vector contains a selectable marker (e.g., ampicillin resistance, hygromycin resistance, tetracycline resistance, kanamycin resistance, or neomycin resistance marker) to facilitate the detection of those cells transformed with desired exogenous DNA sequence.

*Escherichia coli* is an example of a prokaryotic host cell that may be used to clone a subject antibody-encoding polynucleotide. Other microbial hosts suitable for use include bacilli such as *Bacillus subtilis,* and other *enterobacteriaceae* such as *Salmonella, Serratia,* and various *Pseudomonas* species. In these prokaryotic hosts, expression vectors may also be generated, which will typically contain an expression control sequence (e.g., origin of replication) that are compatible with the host cell. In addition, there are many well-known promoters, such as the lactose promoter system, the tryptophan (trp) promoter system, the beta-lactamase promoter system, or the promoter system from bacteriophage lambda. A promoter will typically control the expression, optionally in case of an operator gene sequence, and have ribosome binding site sequence, etc., to initiate and complete transcription and translation.

Other microorganisms, such as yeast, may also be used for expression. Yeast (e.g., S. *cerevisiae)* and Pichia are examples of suitable yeast host cells, and as required a suitable vector has an expression control sequence (e.g., promoter), origin of replication, termination sequence, etc. A typical promoter comprises 3-phosphoglycerate kinase and other saccharolytic enzymes. Particularly, inducible yeast promoters include promoters from ethanol dehydrogenase, isocytochrome C, and enzymes responsible for maltose and galactose utilization

In addition to microorganisms, mammalian cells (e.g., mammalian cells grown in in vitro cell culture) may also be used to express and produce the anti-Tau antibodies of the application (e.g., polynucleotides encoding the subject anti-Tau antibodies). See Winnacker, From Genes to Clones, VCH Publishers, N.Y., N.Y. (1987). Suitable mammalian host cells include CHO cell lines, various Cos cell lines, HeLa cells, myeloma cell lines, and transformed B cells or hybridomas. Expression vectors for use in these cells may comprise expression control sequences such as origin of replication, promoter and enhancer (Queen et al., Immunol. Rev. 89:49 (1986)), and necessary sites for processing information such as ribosome binding sites, RNA splicing sites, polyadenylation sites, and transcription terminator sequences. Examples of suitable expression control sequences are promoters derived from immunoglobulin gene, SV40, adenovirus, bovine papilloma virus, cytomegalovirus, and the like. See Co et al, J. Immunol. 148:1149 (1992).

Once synthesized (chemically or recombinantly), the plasminogen of the present application may be purified according to standard procedures in the art, including ammonium sulfate precipitation, affinity column, column chromatography, high performance liquid chromatography (HPLC), gel electrophoresis, and the like. The plasminogen is substantially pure, e.g., at least about 80-85% pure, at least about 85-90% pure, at least about 90-95% pure, or 98-99% pure or purer, e.g., free of contaminants such as cellular debris, macromolecules other than the target product, and the like.

**Medicament Formulation**

A therapeutic formulation may be prepared by mixing a component of plasminogen activation pathway or a related compound thereof (e.g. plasminogen) of desired purity with an optional pharmaceutical carrier, excipient, or stabilizer (Remington's Pharmaceutical Sciences, 16th edition, Osol, A. ed. (1980)), to form a lyophilized formulation or an aqueous solution. An acceptable carrier, excipient, or stabilizer is non-toxic to a recipient at the employed dosage and concentration, including buffers such as phosphate, citrate and other organic acids; antioxidants such as ascorbic acid and methionine; preservatives (such as octadecyldimethylbenzylammonium chloride; hexanediamine chloride; benzalkonium chloride, benzethonium chloride; phenol, butanol or benzyl alcohol; alkyl parahydroxybenzoate such as methyl or propyl paraben; catechol; resorcinol; cyclohexanol; 3-pentanol; m-cresol); low molecular weight polypeptides (less than about 10 residues); proteins such as serum albumin, gelatin or immunoglobulins; hydrophilic polymers such as polyvinylpyrrolidone; amino acids such as glycine, glutamine, asparagine, histidine, arginine or lysine; monosaccharides, disaccharides and other carbohydrates such as glucose, mannose, or dextrin; chelating agents such as EDTA; carbohydrates such as sucrose, mannitol, fucose, or sorbitol; salt-forming counterions such as sodium; metal complexes (such as zinc-protein complexes); and/or nonionic surfactants such as TWENTM, PLURONICSTM or polyethylene glycol (PEG). Preferred lyophilized anti-VEGF antibody formulation is described in WO 97/04801, which is incorporated herein by reference.

The formulations according to the present application may also contain more than one active compound as required for the particular condition to be treated, preferably those compounds are complementary in activity and do not have side effects with each other.

The plasminogen according to the present application may be encapsulated in microcapsules prepared by techniques such as coacervation or interfacial polymerization, for example, the plasminogen may be placed in colloidal drug delivery systems (e.g., liposomes, albumin microspheres, microemulsions, nanoparticles and nanocapsules) or in hydroxymethyl cellulose or gel-microcapsules and poly-(methyl methacrylate) microcapsules in macroemulsions. These techniques are disclosed in Remington's Pharmaceutical Sciences 16th edition, Osol, A. Ed. (1980).

The component of plasminogen activation pathway or a related compound thereof (e.g. plasminogen) according to the present application for in vivo administration must be sterile. This may be easily achieved by filtration through sterilizing filters before or after lyophilization and reformulation.

The component of plasminogen activation pathway or a related compound thereof (e.g. plasminogen) according to the present application may be prepared as a sustained-release formulation. Suitable examples of sustained-release formulations include semipermeable matrices of solid hydrophobic polymers which have a certain shape and contain glycoprotein, for example, membranes or microcapsules. Examples of sustained-release matrices include polyesters, hydrogels such as poly(2-hydroxyethyl-methacrylate) (Langer et al., J. Biomed. Mater. Res., 15:167-277 (1981); Langer, Chem. Tech., 12:98-105 (1982)), or poly(vinyl alcohol), polylactide (US Pat. No.3,773,919, EP58,481), copolymers of L-glutamic acid and γ-ethyl-L-glutamic acid (Sidman, et al., Biopolymers 22:547 (1983)), non-degradable ethylene-vinyl acetate (Langer, et al., supra), or degradable lactic acid-glycolic acid copolymers such as Lupron Depot™ (injectable microspheres consisting of lactic acid-glycolic acid copolymer and leuprolide acetate), and poly-D-(-)-3-hydroxybutyric acid. Polymers such as ethylene-vinyl acetate and lactic acid-glycolic acid may release molecules continuously for more than 100 days, while some hydrogels release proteins for shorter period of time. Rational strategies to stabilize proteins may be devised based on the relevant mechanisms. For example, if the mechanism of condensation is found to form intermolecular S-S bond through thiodisulfide interchange, then stabilization may be achieved by modifying sulfhydryl residues, lyophilizing from acidic solutions, controlling humidity, using suitable additives, and developing specific polymer matrix composition.

## Administration and Dosage

Administration of the pharmaceutical composition according to the present application may be accomplished by different means, e.g., nasal inhalation, aerosol inhalation, nasal drop or eye drop, intravenous administration, intraperitoneal administration, subcutaneous administration, intracranial administration, intrathecal administration, intraarteral (e.g., via the carotid artery), intramuscular administration, and rectal administration.

Preparations for parenteral administration include sterile aqueous or non-aqueous solutions, suspensions and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, or fixed oils. Intravenous vehicles include fluid and nutritional supplements, electrolyte supplements, and the like. Preservatives and other additives may also be present, such as, for example, antimicrobials, antioxidants, chelating agents, and inert gases, etc.

Dosing regimens will be determined by medical personnel based on various clinical factors. As is well known in the medical field, the dosage for any patient depends on a variety of factors, including the patient's size, body surface area, age, the particular compound to be administered, sex, number and route of administration, general health, and other concomitantly administered medicaments. The dosage range of the pharmaceutical composition comprising the plasminogen according to the present application may be, for example, about 0.0001-2000 mg/kg, or about 0.001-500 mg/kg (e.g., 0.02 mg/kg, 0.25 mg/kg, 0.5 mg/kg, 0.75 mg/kg, 10 mg/kg, 50 mg/kg, etc.) body weight of the subject per day. For example, the dose may be 1 mg/kg body weight, or 50 mg/kg body weight, or in the range of 1-50 mg/kg, or at least 1 mg/kg. Dosages above or below this exemplary range are also contemplated, especially in view of the factors set forth above. Intermediate doses within the above ranges are also included within the scope of the present application. Subjects may be administered such doses daily, every other day, weekly, or according to any other schedule determined by empirical analysis. An exemplary dosage schedule includes 0.01-100 mg/kg on consecutive days. Real-time evaluation of therapeutic efficacy and safety is required during the administration of the medicament of the present application.

## Product or Kit

One embodiment of the present application relates to a product or kit comprising a component of plasminogen activation pathway or a related compound thereof (e.g. plasminogen). The product preferably comprises a container, and a label or package insert. Suitable containers are bottles, vials, syringes, etc. The container may be made of various materials such as glass or plastic. The container contains a composition which is effective for treatment of the disease or condition according to the present application and has a sterile access port (e.g., the container may be an intravenous solution pack or vial containing a stopper penetrable by a hypodermic needle). At least one active agent in the composition is a component of plasminogen activation pathway or a related compound thereof (e.g. plasminogen). The label on or attached to the container indicates that the composition is used for treatment of the diseases mentioned in the present application. The product may further comprise a second container containing a pharmaceutically acceptable buffer, such as phosphate buffered saline, Ringer's solution, and glucose solution. It may further contain other materials required from a commercial and user standpoint, including other buffers, diluents, filters, needles and syringes. In addition, the product comprises a package insert with instructions for use, including, for example, instructing the user of the composition to administrate the composition comprising the component of plasminogen activation pathway or a related compound thereof (e.g. plasminogen) to the patient along with other medicaments for treatment of concomitant diseases.

## EXAMPLE

Human plasminogen used in the following examples is derived from plasma of a human donor, based on methods described in: Kenneth C Robbins, Louis Summaria, David Elwyn et al. Further Studies on the Purification and Characterization of Human Plasminogen and Plasmin. Journal of Biological Chemistry, 1965, 240(1): 541-550; Summaria L, Spitz F, Arzadon L et al. Isolation and characterization of the affinity chromatography forms of human Glu-and Lys-plasminogens and plasmins. J Biol Chem. 1976 Jun 25;251(12):3693-9; HAGAN JJ, ABLONDI FB, DE RENZO EC. Purification and biochemical properties of human plasminogen. J Biol Chem. 1960 Apr; 235:1005-10, with process optimization, being purified from plasma of a human donor, with >98% human plasminogen monomer.

**Example 1. Plasminogen promotes regeneration of myelin sheaths in corpus callosum of bis(cyclohexanone) oxaldihydrazone induced demyelination model mice**

Twenty 8-week-old C57 male mice are randomly divided into two groups: 6 mice in the blank control group and 14 mice in the model group. The mice in the blank control group are fed with normal maintenance diet (purchased from Beijing Keao Xieli Feed Co., Ltd.), and the mice in the model group are fed with modeling diet with 0.2% bis(cyclohexanone) oxaldihydrazone (cuprizone) (Trophic Animal Feed High-Tech Co.,Ltd) for 6 weeks to induce model mice of demyelination[1]. 6 weeks later, mice in the model group are further divided into two groups (plasminogen group and vehicle group) randomly according to body weight, with 7 mice in each group. The mice in the plasminogen group are injected with plasminogen at 1 mg/0.1 ml/day through the tail vein, and the mice in the vehicle group are injected with the same volume of PBS through the tail vein, and the mice in the blank control group are not treated with plasminogen, the treatment is lasted for 14 days. During the administration period, all mice are fed with normal maintenance diet. The day for the start of administration is set as day 1, and the mice are sacrificed on day 15 and their brains are dissected and fixed in 4% paraformaldehyde, dehydrated and embedded. The fixed tissue samples are dehydrated with ethanol gradient and cleared with xylene before being embedded in paraffin. The thickness of the coronal section of the brain tissue is 3 μm, the tissue sections are then dewaxed to water and stained with myelin staining solution for LFB staining. The sections are dehydrated with ethanol gradient and cleared with xylene before sealed with Neutral Balsam. Then they are observed and photographed under an optical microscope.

The results show that the morphology of corpus callosum myelin sheath is basically normal in the blank control group (Figure 1A), and the positive staining of corpus callosum myelin sheath (marked by arrows) is significantly more in the plasminogen group (Figure 1C) than that in the vehicle group (Figure 1B), and the statistical difference is significant (Figure 1D) (* indicates P<0.05). It indicates that plasminogen can promote the regeneration of myelin sheath in bis(cyclohexanone) oxaldihydrazone induced demyelination model mice.

**Example 2 Plasminogen promotes the expression of PLP in corpus callosum of bis(cyclohexanone) oxaldihydrazone induced model mice of demyelination**

Twenty 8-week-old C57 male mice are randomly divided into two groups: 6 mice in the blank control group and 14 mice in the model group. The mice in the blank control group are fed with normal maintenance diet (purchased from Beijing Keao Xieli Feed Co., Ltd.), and the mice in the model group are fed with modeling diet with 0.2% bis(cyclohexanone) oxaldihydrazone (Trophic Animal Feed High-Tech Co. Ltd) for 6 weeks to induce model mice of demyelination [1]. 6 weeks later, mice in the model group are further divided into two groups (plasminogen group and vehicle group) randomly according to body weight, with 7 mice in each group. The mice in the plasminogen group are injected with plasminogen at 1 mg/0.1 ml/day through the tail vein, the mice in the vehicle group are injected with the same volume of PBS through the tail vein, and the mice in the blank control group are not treated with plasminogen, the treatment is lasted for 3 days. During the administration period, all mice are fed with normal maintenance diet. The day for the start of administration is set as day 1, and the mice are sacrificed on day 4 and their brains are dissected and fixed in 4% paraformaldehyde, dehydrated and embedded. The fixed tissue samples are dehydrated with ethanol gradient and cleared with xylene before being embedded in paraffin. The thickness of the coronal section of the brain tissue is 3 μm. After the tissue sections are dewaxed and rehydrated, washing them once with water, repairing with citric acid for 30 minutes, then cooling at room temperature for 10 minutes and rinsing gently with water. The tissue sections are incubated with 3% hydrogen peroxide for 15 min, circling the tissues with a PAP pen; blocking with 10% goat serum (Vector laboratories, Inc., USA) for 1 h, the goat serum is discarded when the time is up. The tissue sections are incubated in rabbit-derived anti-proteolipid protein (PLP) antibody (Abcam) overnight at 4°C, washing twice with PBS for 5 min each time; then incubating in goat anti-rabbit IgG (HRP) antibody (Abcam) secondary antibody at room temperature for 1 hour, and washing twice with PBS for 5 minutes each time. The color is developed according to the DAB kit (Vector laboratories, Inc., USA), after washing with water for 3 times, counterstaining with hematoxylin for 30 seconds, returning to blue under running water for 5 min, and then washing once with PBS. After gradient dehydration, the sections are subjected to being cleared and sealed, finally observing under a 200× optical microscope.

Proteolipid protein (PLP) is a highly hydrophobic membrane protein and is the most abundant myelin lipid in the central nervous system [2].

The results show that the expression of PLP in the corpus callosum (marked by arrows) is significantly more in the plasminogen group (Figure 2C) than that in the vehicle group (Figure 2B); Comparing to the vehicle group, the expression in the plasminogen group is more similar to that of the mice in the blank control group (Figure 2A). It indicates that plasminogen promotes the expression of corpus callosum PLP and promotes myelin sheath regeneration in bis(cyclohexanone) oxaldihydrazone induced demyelination model mice.

**Example 3 Plasminogen promotes the expression of neurofilament protein (NFP) in corpus callosum of bis(cyclohexanone) oxaldihydrazone induced model mice of demyelination**

Twenty 8-week-old C57 male mice are randomly divided into two groups: 6 mice in the blank control group and 14 mice in the model group. The mice in the blank control group are fed with normal maintenance diet (purchased from Beijing Keao Xieli Feed Co., Ltd.), and the mice in the model group are fed with modeling diet with 0.2% bis(cyclohexanone) oxaldihydrazone (Trophic Animal Feed High-Tech Co. Ltd) for 6 weeks to induce model mice of demyelination [1]. 6 weeks later, mice in the model group are further divided into two groups (plasminogen group and vehicle group) randomly according to body weight, with 7 mice in each group. The mice in the plasminogen group are injected with plasminogen at 1 mg/0.1 ml/day through the tail vein, the mice in the vehicle group are injected with the same volume of PBS through the tail vein, and the mice in the blank control group are not treated with plasminogen, the treatment is lasted for 14 days. During the administration period, all mice are fed with normal maintenance diet. The day for the start of administration is set as day 1, and the mice are sacrificed on day 15 and their brains are dissected and fixed in 4% paraformaldehyde, dehydrated and embedded. The fixed tissue samples are dehydrated with ethanol gradient and cleared with xylene before being embedded in paraffin. The thickness of the coronal section of the brain tissue is 3 $\mu$m. After the tissue sections are dewaxed and rehydrated, washing them once with water, repairing with citric acid for 30 minutes, then cooling at room temperature for 10 minutes and rinsing gently with water. Then tissue sections are incubated with 3% hydrogen peroxide for 15 min, circling the tissues with a PAP pen; blocking with 10% goat serum (Vector laboratories, Inc., USA) for 1 h, the goat serum solution is discarded when the time is up. The tissue sections are incubated in rabbit-derived anti-NFP antibody (Abcam, ab207176) overnight at 4°C, washing twice with PBS for 5 min each time; then incubating in goat anti-rabbit IgG (HRP) antibody (Abcam) secondary antibody at room temperature for 1 hour, and washing twice with PBS for 5 minutes each time. The color is developed according to the DAB kit (Vector laboratories, Inc., USA), after washing with water for 3 times, counterstaining with hematoxylin for 30 seconds, returning to blue under running water for 5 min, and then washing once with PBS. After gradient dehydration, the sections are subjected to being cleared and sealed, finally observing under a 200× optical microscope.

Neurofilament protein (NFP) is a protein that forms the intermediate filaments of nerve cell axons. Its function is to provide elasticity to allow nerve fibers to stretch easily and prevent breakage, and it is very important in maintaining the cytoskeleton, stabilizing cell morphology and axonal transport [3].

The results show that the expression of NFP in the corpus callosum of mice in the plasminogen group (Figure 3C) (marked by arrows) is significantly more than that in the vehicle group (Figure 3B), and the statistical difference is significant (* for P<0.05) (Figure 3D); Comparing to that in the vehicle group, the expression of NFP in the corpus callosum of the plasminogen group is more similar to that in the blank control group (Figure 3A). It indicates that plasminogen can promote the expression of NFP, thereby promoting regeneration of nerve fiber.

**Example 4 Plasminogen promotes increased expression of neurofilament protein in the corpus callosum of multiple sclerosis model mice**

30 female C57 mice are weighed before constructing the model, and after excluding the abnormal mice according to body weight, all mice are randomly divided into two groups, 8 mice in the blank control group and 22 mice in the model group. After grouping, mice in the blank control group are fed with normal maintenance diet (purchased from Beijing Keao Xieli Feed Co., Ltd), and mice in the model group are fed with modeling diet containing 0.6% bis(cyclohexanone) oxaldihydrazone (CPZ) (manufacturer: Shanghai Yuanye Bio-Technology Co., Ltd., item no. S30349) for 42 days to induce multiple sclerosis [1]. After completion of modeling, all mice are tested in the open field experiment, and mice in the model group are grouped according to the test results, 11 mice in the vehicle group and 11 mice in the plasminogen group. After the grouping is completed, administration to all mice is started on day 1. The mice in the blank control group and the mice in the vehicle group are injected with the vehicle at 0.1 ml/day per mouse through the tail vein, and the mice in the plasminogen group are injected with plasminogen at 1 mg/day per mouse through the tail vein. The treatment is lasted for 35 days. During the administration period, all mice are fed with normal maintenance diet. The mice are sacrificed on day 36, and the brain tissues are fixed in 10% formaldehyde solution, dehydrated and embedded. The fixed tissues are dehydrated with ethanol gradient and cleared with xylene, and then embedded in paraffin. Brain tissue is coronally sectioned at a thickness of 3 $\mu$m, and the sections are dehydrated and embedded. The fixed tissue samples are dehydrated with ethanol gradient and cleared with xylene before being embedded in paraffin. Brain tissue is coronally sectioned at a thickness of 3 $\mu$m. After the tissue sections are dewaxed and rehydrated, washing them once with water, repairing with citric acid for 30 minutes, then cooling at room temperature for 10 minutes and rinsing gently with water.

The tissue sections are then incubated with 3% hydrogen peroxide for 15 min, circling the tissues with a PAP pen; blocking with 10% goat serum (Vector laboratories, Inc., USA) for 1 h, the goat serum solution is discarded when the time is up. The tissue sections are incubated in rabbit-derived anti-NFP antibody (Abcam, ab207176) overnight at 4°C, washing twice with PBS for 5 min each time; then incubating in goat anti-rabbit IgG (HRP) antibody (Abcam) secondary antibody at room temperature for 1 hour, and washing twice with PBS for 5 minutes each time. The color is developed according to the DAB kit (Vector laboratories, Inc., USA), after washing with water for 3 times, counterstaining with hematoxylin for 30 seconds, returning to blue under running water for 5 min, and then wash once with PBS. After gradient dehydration, the sections are subjected to being cleared and sealed, finally observing under a 100× optical microscope.

The results show that the corpus callosum of the blank control group (Figure 4A) expresses a certain level of NFP (marked by arrow), the expression of NFP in the corpus callosum of the mice in the vehicle group (Figure 4B) is significantly reduced, and the level of NFP expression in the corpus callosum of the mice in the plasminogen group (Figure 4C) is significantly higher than that in the mice of the vehicle group, and the statistical difference is significant (* indicates $P<0.05$) (Figure 4D). This result suggests that plasminogen can promote the expression of NFP in corpus callosum of multiple sclerosis model mice.

**Example 5 Plasminogen promotes increased levels of corpus callosum proteolipid protein in multiple sclerosis model mice**

30 female C57 mice are weighed before modeling, and after excluding the abnormal mice according to body weight, all mice are randomly divided into two groups, 8 mice in the blank control group and 22 mice in the model group. After grouping, mice in the blank control group are fed with maintenance diet, and mice in the model group are fed with modeling diet containing 0.6% bis(cyclohexanone) oxaldihydrazone (CPZ) (manufacturer: Shanghai Yuanye Bio-Technology Co., Ltd., item no. S30349) for 42 days to induce multiple sclerosis model [1]. After completion of modeling, all mice are tested in the open field experiment, and mice in the model group are grouped according to the test results, 11 mice in the vehicle group and 11 mice in the plasminogen group. After the grouping is completed, administration to all mice is started on day 1. The mice in the blank control group and the mice in the vehicle group are injected with the vehicle at 0.1 ml/day per mouse through tail vein, and the mice in the plasminogen group are injected with plasminogen at 1 mg/day per mouse through tail vein. The treatment is lasted for 35 days. During the administration period, all mice are fed with normal maintenance diet (purchased from Beijing Keao Xieli Feed Co., Ltd). The mice are sacrificed on day 36, and the brain tissues are fixed in 10% formaldehyde solution, dehydrated and embedded. The fixed tissue samples are dehydrated with ethanol gradient and cleared with xylene, and then embedded in paraffin. Brain tissue is coronally sectioned at a thickness of 3 μm. After the tissue sections are dewaxed and rehydrated, washing them once with water, repairing with citric acid for 30 minutes, then cooling at room temperature for 10 minutes and rinsing gently with water. The tissue sections are then incubated with 3% hydrogen peroxide for 15 min, circling the tissues with a PAP pen; blocking with 10% goat serum (Vector laboratories, Inc., USA) for 1 h, the goat serum solution is discarded when the time is up. The tissue sections are incubated in rabbit-derived anti-PLP antibody (Abcam) overnight at 4°C, washing twice with PBS for 5 min each time; then incubating in goat anti-rabbit IgG (HRP) antibody (Abcam) secondary antibody at room temperature for 1 hour, and washing twice with PBS for 5 minutes each time. The color is developed according to DAB kit (Vector laboratories, Inc., USA), after washing with water for 3 times, counterstaining with hematoxylin for 30 seconds, returning to blue under running water for 5 min, and then wash once with PBS. After gradient dehydration, the sections are subjected to being cleared and sealed, finally observing under a 100× optical microscope.

The results show that the corpus callosum of the blank control group (Figure 5A) expresses a certain level of PLP (marked by arrows), the expression of PLP in the corpus callosum of the mice in the vehicle group (Figure 5B) is significantly reduced, and the expression level of PLP in the corpus callosum of the mice in the plasminogen group (Figure 5C) is significantly higher than that of the mice in the vehicle group. This result suggests that plasminogen can promote the expression of PLP in the corpus callosum of multiple sclerosis model mice.

**Example 6 Plasminogen promotes the increase of MBP level in the corpus callosum of multiple sclerosis model mice**

30 female C57 mice are weighed before modeling. After excluding the abnormal mice according to body weight, all mice are randomly divided into two groups, 8 mice in the blank control group and 22 mice in the model group. After grouping, mice in the blank control group are fed with normal maintenance diet (purchased from Beijing Keao Xieli Feed Co., Ltd), and mice in the model group are fed with modeling diet containing 0.6% bis(cyclohexanone) oxaldihydrazone (CPZ) (manufacturer: Shanghai Yuanye Bio-Technology Co., Ltd., item no. S30349) for 42 days to induce multiple sclerosis [1]. After completion of modeling, all mice are tested in the open field experiment, and mice in the model group are grouped according to the test results, 11 mice in the vehicle group and 11 mice in the plasminogen group. After the grouping is completed, administration to all mice is started on day 1. The mice in the blank control group and the mice in the vehicle group are injected with the vehicle at 0.1 ml/day per mouse through tail vein, and the mice in the plasminogen group are injected with plasminogen at 1 mg/day per mouse through tail vein. The treatment is lasted for 35 days. During the administration period, all mice are fed with normal maintenance diet. The mice are sacrificed on day 36, and the brain tissues are fixed in 10% formaldehyde solution, dehydrated and embedded. The fixed tissues are dehydrated with ethanol gradient and cleared with xylene, and then embedded in paraffin. Brain tissue is coronally sectioned at a thickness of 3 $\mu$m, and sections are dewaxed and rehydrated, washing them once with water, repairing with citric acid for 30 minutes, then cooling at room temperature for 10 minutes and rinsing gently with water. The tissue sections are then incubated with 3% hydrogen peroxide for 15 min, circling the tissues with a PAP pen; blocking with 10% goat serum (Vector laboratories, Inc., USA) for 1 h, the goat serum solution is discarded when the time is up. The tissue sections are incubated in rabbit-derived anti-myelin basic protein (MBP) antibody (Abcam) overnight at 4°C, washing twice with PBS for 5 min each time; then incubating in goat anti-rabbit IgG (HRP) antibody (Abcam) secondary antibody at room temperature for 1 hour, and washing twice with PBS for 5 minutes each time. The color is developed according to DAB kit (Vector laboratories, Inc., USA), after washing with water for 3 times, counterstaining with hematoxylin for 30 seconds, returning to blue under running water for 5 min, and then wash once with PBS. After gradient dehydration, the sections are subjected to being cleared and sealed, finally observing under a 100$\times$ optical microscope.

Myelin basic protein (MBP) is a strongly basic membrane protein synthesized by oligodendrocytes in the central nervous system and Schwann cells in the peripheral nervous system of vertebrates. It contains a variety of basic amino acids.

The results show that the corpus callosum of the blank control group (Figure 6A) expresses a certain level of MBP (marked by arrows), and MBP in the corpus callosum of the mice in the plasminogen group (Figure 6C) is significantly higher than that of the mice in the vehicle group (Figure 6B), and the statistical difference is close to significant (P=0.063) (Figure 6D). This result suggests that plasminogen can promote the increasing of MBP level in the corpus callosum of multiple sclerosis model mice.

**Example 7 Plasminogen promotes repair of hippocampal injury inflammation in multiple sclerosis model mice**

30 female C57 mice are weighed before modeling. After excluding abnormal mice according to body weight, all mice are randomly divided into two groups, 8 mice in the blank control group and 22 mice in the model group. After grouping, mice in the blank control group are fed with normal maintenance diet (purchased from Beijing Keao Xieli Feed Co., Ltd), and mice in the model group are fed with modeling diet containing 0.6% bis(cyclohexanone) oxaldihydrazone (CPZ) (manufacturer: Shanghai Yuanye Bio-Technology Co., Ltd., item no. S30349) for 42 days to induce multiple sclerosis [1]. After completion of modeling, all mice are tested in the open field experiment, and mice in the model group are grouped according to the test results, 11 mice in the vehicle group and 11 mice in the plasminogen group. After the grouping is completed, administration to all mice is started on day 1. The mice in the blank control group and the mice in the vehicle group are injected with the vehicle at 0.1 ml/day per mouse through tail vein, and the mice in the plasminogen group are injected with plasminogen at 1 mg/day per mouse through tail vein. The treatment is lasted for 35 days. During the administration period, all mice are fed with normal maintenance diet. The mice are sacrificed on day 36, and the brain tissues are fixed in 10% formaldehyde solution, dehydrated and embedded. The fixed tissues are dehydrated with ethanol gradient and cleared with xylene before being embedded in paraffin. Brain tissue is coronally sectioned at a thickness of 3 $\mu$m, and the sections are dewaxed and rehydrated, washing once with water, repairing with citric acid for 30 minutes, then cooling at room temperature for 10 minutes and rinsing gently with water. The tissue sections are then incubated with 3% hydrogen peroxide for 15 min, circling the tissues with a PAP pen; blocking with 10% goat serum (Vector laboratories, Inc., USA) for 1 h; the goat serum solution is discarded when the time is up. The tissue sections are incubated in rabbit-derived anti-NFP antibody (Abcam, ab207176) overnight at 4°C, washing twice with PBS for 5 min each time; then incubating in goat anti-rabbit IgG (HRP) antibody (Abcam) secondary antibody at room temperature for 1 hour, and washing twice with PBS for 5 minutes each time. The color is developed according to DAB kit (Vector laboratories, Inc., USA), after washing with water for 3 times, counterstaining with hematoxylin for 30 seconds, returning to blue under running water for 5 min, and then wash once with PBS. After gradient dehydration, the sections are subjected to being cleared and sealed, finally observed and photographed under a 200$\times$ optical microscope, and the photographed images are analyzed by Imaging-Pro software for optical density of positive staining.

Ionized calcium binding adaptor molecule-1 (Iba-1) is a microglia surface marker in the central nervous system. Microglia are immune cells in the central nervous system that rapidly sense neurological deficits and are activated in response to lesions or injury. Activated microglia show significant changes in number and morphology and migrate to the site of injury, performing various functions such as phagocytosis of dead cells and promotion of increased production of inflammatory cytokines et al [4].

The results show that a certain amount of microglia (marked by arrows) are present in the hippocampi of mice in the blank control group (Figure 7A), and the amount of microglia in the hippocampi of mice in the vehicle group (Figure 7B) is significantly increased, but the number of microglia in the hippocampi of mice in the plasminogen group is significantly more than that in the vehicle group (Figure 7C), and the statistical differences are significant (Figure 7D) (* indicates P<0.05,*** indicates P<0.001). It suggests that plasminogen can promote repair of hippocampi injury inflammation in multiple sclerosis model mice.

**Example 8 Plasminogen promotes the increase of hippocampal BDNF level in multiple sclerosis model mice**

30 female C57 mice are weighed before modeling, and after excluding the abnormal mice according to body weight, all mice are randomly divided into two groups, 8 mice in the blank control group and 22 mice in the model group. After grouping, mice in the blank control group are fed with normal maintenance diet (purchased from Beijing Keao Xieli Feed Co., Ltd), and mice in the model group are fed with modeling diet containing 0.6% bis(cyclohexanone) oxaldihydrazone (CPZ) (manufacturer: Shanghai Yuanye Bio-Technology Co., Ltd., item no. S30349) for 42 days to induce multiple sclerosis [1]. After completion of modeling, all mice are tested in the open field experiment, and mice in the model group are grouped according to the test results, 11 mice in the vehicle group and 11 mice in the plasminogen group. After the grouping is completed, administration to all mice is started on day 1. The mice in the blank control group and the mice in the vehicle group are injected with the vehicle at 0.1 ml/day per mouse through tail vein, and the mice in the plasminogen group are injected with plasminogen at 1 mg/day per mouse through tail vein. The treatment is lasted for 35 days. During the administration period, all mice are fed with normal maintenance diet. The mice are sacrificed on day 36, and the brain tissues are fixed in 10% formaldehyde solution, dehydrated and embedded. The fixed tissues are dehydrated with ethanol gradient and cleared with xylene before being embedded in paraffin. Brain tissue is coronally sectioned at a thickness of 3 $\mu$m, and sections are dewaxed and rehydrated and then washed once with water, repairing with citric acid for 30 minutes, then cooling at room temperature for 10 minutes and rinsing gently with water. The tissue sections are then incubated with 3% hydrogen peroxide for 15 min, circling the tissue with a PAP pen; blocking with 10% goat serum (Vector laboratories, Inc., USA) for 1 h; the goat serum solution is discarded when the time is up. The tissue sections are incubated in rabbit-derived anti- brain-derived neurotrophic factor (BDNF) antibody (BosterBio, PB9075) overnight at 4°C, washing twice with PBS for 5 min each time; then incubating in goat anti-rabbit IgG (HRP) antibody (Abcam) secondary antibody at room temperature for 1 hour, and washing twice with PBS for 5 minutes each time. The color is developed according to DAB kit (Vector laboratories, Inc., USA), after washing with water for 3 times, counterstaining with hematoxylin for 30 seconds, returning to blue under running water for 5 min, and then wash once with PBS. After gradient dehydration, the sections are subjected to being cleared and sealed, and the sections are observed and photographed under a 400× optical microscope, and the photographed images are analyzed by Imaging-Pro software for optical density of positive staining.

Mature brain-derived neurotrophic factor (BDNF) and its receptors are widely distributed in the central nervous system and play an important role in neuronal survival, differentiation, growth and development during the development of the central nervous system, and can prevent neuronal death by injury, improve the pathological state of neurons, and promote the biological effects of injured neuronal regeneration and differentiation, and is necessary for the maintenance of survival and normal physiological function of neurons in the mature central and peripheral nervous system[5].

The results show that the hippocampi of mice in the blank control group (Figure 8A) have a certain level of BDNF (marked by arrows), the hippocampi of mice in the vehicle group (Figure 8B) have an increased level of BDNF, and the level of BDNF in the hippocampi of mice in the plasminogen group is significantly higher than that in the vehicle group (Figure 8C), and the statistical difference is close to significant (Figure 8D) (P=0.095). It suggests that plasminogen can promote the increase of hippocampus BDNF level in multiple sclerosis model mice.

**Example 9 Plasminogen promotes increased hippocampal astrocyte activity in multiple sclerosis model mice**

30 female C57 mice are weighed before modeling, and after excluding the abnormal mice according to body weight, all mice are randomly divided into two groups, 8 mice in the blank control group and 22 mice in the model group. After grouping, mice in the blank control group are fed with normal maintenance diet (purchased from Beijing Keao Xieli Feed Co., Ltd), and mice in the model group are fed with modeling diet containing 0.6% bis(cyclohexanone) oxaldihydrazone (CPZ) (manufacturer: Shanghai Yuanye Bio-Technology Co., Ltd., item no. S30349) for 42 days to induce multiple sclerosis model [1]. After completion of modeling, all mice are tested in the open field experiment, and mice in the model group are grouped according to the test results, 11 mice in the vehicle group and 11 mice in the plasminogen group. After the grouping is completed, administration to all mice is started on day 1. The mice in the blank control group and the mice in the vehicle group are injected with the vehicle at 0.1 ml/day per mouse through tail vein, and the mice in the plasminogen group are injected with plasminogen at 1 mg/day per mouse through tail vein. The treatment is lasted for 35 days. During the administration period, all mice are fed with normal maintenance diet. The mice are sacrificed on day 36, and the brain tissues are fixed in 10% formaldehyde solution, dehydrated and embedded. The fixed tissues are dehydrated with ethanol gradient and cleared with xylene before being embedded in paraffin. Brain tissue is coronally sectioned at a thickness of 3 $\mu$m, and sections are dewaxed and rehydrated and then washed once with water, repairing with citric acid for 30 minutes, then cooling at room temperature for 10 minutes and rinsing gently with water. The tissue sections are then incubated with 3% hydrogen peroxide for 15 min, circling the tissues with a PAP pen; blocking with 10% goat serum (Vector laboratories, Inc., USA) for 1 h; the goat serum solution is discarded when the time is up. The tissue sections are incubated in rabbit-derived anti- glial fibrillary acidic protein (GFAP) antibody (Abcam, ab4648) overnight at 4°C, washing twice with PBS for 5 min each time; then incubating in goat anti-rabbit IgG (HRP) antibody (Abcam) secondary antibody at room temperature for 1 hour, and washing twice with PBS for 5 minutes each time. The color is developed according to DAB kit (Vector laboratories, Inc., USA), after washing with water for 3 times, counterstaining with hematoxylin for 30 seconds, returning to blue under running water for 5 min, and then wash once with PBS. After gradient dehydration, the sections are subjected to being cleared and sealed, and the sections are observed and photographed under a 200× optical microscope, and the photographed images are analyzed by Imaging-Pro software for optical density of positive staining.

Glial fibrillary acidic protein (GFAP) is a signature intermediate filament protein in astrocytes that is involved in cytoskeleton composition and maintains its tension strength [6].

The results show that the hippocampi of mice in the blank control group (Figure 9A) express a certain level of GFAP (marked by arrows), the hippocampi of mice in the vehicle group (Figure 9B) have a decreased expression of GFAP, and the expression of GFAP in the hippocampi of mice in the plasminogen group is significantly higher than that in the vehicle group (Figure 9C), and the statistical difference is close to significant (Figure 9D) (P=0.051). It suggests that plasminogen can promote the increase of GFAP expression in the hippocampi and astrocyte activity in multiple sclerosis model mice.

**Example 10 Plasminogen alleviates depressive behavior in multiple sclerosis model mice**

30 female C57 mice are weighed before modeling, and after excluding the abnormal mice according to body weight, all mice are randomly divided into two groups, 8 mice in the blank control group and 22 mice in the model group. After grouping, mice in the blank control group are fed with normal maintenance diet (purchased from Beijing Keao Xieli Feed Co., Ltd), and mice in the model group are fed with modeling diet containing 0.6% bis(cyclohexanone) oxaldihydrazone (CPZ) (manufacturer: Shanghai Yuanye Bio-Technology Co., Ltd., item no. S30349) for 42 days to induce multiple sclerosis [1]. After completion of modeling, all mice are tested in the open field experiment, and mice in the model group are grouped according to the test results, 11 mice in the vehicle group and 11 mice in the plasminogen group. After the grouping is completed, administration to all mice is started on day 1. The mice in the blank control group and the mice in the vehicle group are injected with the vehicle at 0.1 ml/day per mouse through tail vein, and the mice in the plasminogen group are injected with plasminogen at 1 mg/day per mouse through tail vein. The treatment is lasted for 28 days. During the administration period, all mice are fed with normal maintenance diet. An open field experiment is performed on day 29.

Patients with multiple sclerosis often have psychiatric symptoms, mostly depression, irritability and bad temper, some patients show euphoria, excitement, but also apathy, somnolence, forced crying and forced laughing, unresponsiveness, low intelligence, repetitive language, suspicion and persecutory delusion. Cognitive dysfunction such as hypomnesis and attention impairment may also occur [7].

Open field experiment

During the experiment, mice are placed in the center of the bottom surface of the open field (40x40x40cm), simultaneously videotaped and timed, and observed for a duration of 5 minutes, with 3 tests per mouse. The Smart system is a complete and easy to use video tracking system for evaluating the behavior of the experimental animals. It allows recording trajectories, activities, specific behaviors (such as rotation, stretching and rearing) and events, and performing calculations of various analytical parameters. In this experiment, the Smart3.0 system is used to record and analyze the movements of mice with parameters such as total movement distance, percentage of resting time in the boundary zone, average speed of movement in the central zone and average speed of movement in the boundary zone. 70% alcohol is used to wipe the box in each experiment to prevent the preference produced by olfactory [8].

The design principle of the open field experiment is based on the phobotaxis of mice, which refers to the fear of mice of open, unknown and potentially dangerous places, and thus they have natural tendency to move "against the wall". Phobotaxis is evaluated by the activity of mice in the perimeter zone (four corners and four sides) of the open field. Judging from the time spent in the peripheral zone which reflects phobotaxis, the mouse is more "adventurous" when the time decreases. A significant increase in activity time in the central zone indicates lower levels of phobotaxis and anxiety (depression). Total movement distance is the total movement distance of the mice in each area during the open field experiment. The results show that the mice in the blank control group have a certain total movement distance, and the mice in the vehicle group have a significantly increased total movement distance. The total movement distance of the mice in the plasminogen group is significantly less than that in the vehicle group, the statistical difference is significant

(* indicates P<0.05) (Figure 10), and it is similar to that in the blank control group. It indicates that plasminogen can alleviate the depressive behavior of multiple sclerosis model mice to some extent.

**Example 11 Plasminogen alleviates depressive behavior in multiple sclerosis model mice**

30 female C57 mice are weighed before modeling, and after excluding the abnormal mice according to body weight, all mice are randomly divided into two groups, 8 mice in the blank control group and 22 mice in the model group. After grouping, mice in the blank control group are fed with normal maintenance diet (purchased from Beijing Keao Xieli Feed Co., Ltd), and mice in the model group are fed with modeling diet containing 0.6% bis(cyclohexanone) oxaldihydrazone (CPZ) (manufacturer: Shanghai Yuanye Bio-Technology Co., Ltd., item no. S30349) for 42 days to induce multiple sclerosis [1]. After completion of modeling, all mice are tested in the open field experiment, and mice in the model group are grouped according to the test results, 11 mice in the vehicle group and 11 mice in the plasminogen group. After the grouping is completed, administration to all mice is started on day 1. The mice in the blank control group and the mice in the vehicle group are injected with the vehicle at 0.1 ml/day per mouse through tail vein, and the mice in the plasminogen group are injected with plasminogen at 1 mg/day per mouse through tail vein. The treatment is lasted for 28 days. During the administration period, all mice are fed with normal maintenance diet. An open field experiment is performed on day 29.

The percentage of resting time in the boundary zone = resting time in the boundary zone/total observation time. The results show that the mice in the blank control group have a certain percentage of resting time in the boundary zone, which is about 57.8%; the percentage of resting time in the boundary zone of the mice in the vehicle group is significantly lower, which is about 49.3%, and the percentage of resting time in the boundary zone of the mice in the plasminogen group is about 58.4%, which is significantly greater than that in the vehicle group, and the statistical difference is significant (* indicates P<0.05) (Figure 11) and it is similar to that in the blank control group. It indicates that plasminogen can alleviate the depressive behavior of multiple sclerosis model mice to some extent.

**Example 12 Plasminogen improves social memory in multiple sclerosis model mice**

30 female C57 mice are weighed before modeling, and after excluding the abnormal mice according to body weight, all mice are randomly divided into two groups, 8 mice in the blank control group and 22 mice in the model group. After grouping, mice in the blank control group are fed with normal maintenance diet (purchased from Beijing Keao Xieli Feed Co., Ltd), and mice in the model group are fed with modeling diet containing 0.6% bis(cyclohexanone) oxaldihydrazone (CPZ) (manufacturer: Shanghai Yuanye Bio-Technology Co., Ltd., item no. S30349) for 42 days to induce multiple sclerosis [1]. After completion of modeling, all mice are tested in the open field experiment, and mice in the model group are grouped according to the test results, 11 mice in the vehicle group and 11 mice in the plasminogen group. After the grouping is completed, administration to all mice is started on day 1. The mice in the blank control group and the mice in the vehicle group are injected with the vehicle at 0.1 ml/day per mouse through tail vein, and the mice in the plasminogen group are injected with plasminogen at 1 mg/day per mouse through tail vein. The treatment is lasted for 34 days. During the administration period, all mice are fed with normal maintenance diet. A three-chamber sociability test is performed on day 35.

Before the start of the three-chamber test experiment, the mice are acclimatized in the behavioral test room for half an hour; the three boxes are separated by a transparent glass resin plate, and the test mice are acclimatized in the middle box for 5 minutes; the unfamiliar mouse 1 is randomly placed in the metal cage in the left or right box, and the metal cage in the other box is left empty; the glass resin plate separating the boxes is removed so that the test mouse can move freely in the three boxes for 10 minutes; the videotaping is started immediately and relevant parameters are recorded: Phase I: 1) the number and duration of direct contact between the experimental mouse and the unfamiliar mouse 1 or the empty metal cage, 3-5 cm around the metal cage is defined as the contact range; 2) the number and duration of the experimental mouse entering each box, and the mouse is considered to be in a box when both head and 4 paws are in that box. Phase 2: In the second phase of the experiment, a second unfamiliar mouse (unfamiliar mouse 2) is placed into the empty metal cage, and then the duration and number of contacts between the experimental mouse and unfamiliar mouse 1 and 2 are recorded for ten minutes. Normal mice exhibit social behavior, and in phase I of the experiment, normal mice usually interact with unfamiliar mouse 1 for significantly longer time and more often than with the empty metal cage. Also, mice have memory and "abandon the old for the new", therefore in phase 2, mice prefer to interact with a previously unseen unfamiliar mouse 2 rather than unfamiliar mouse 1 with whom they have already communicated for 10 minutes.

Percentage of resting time = resting time/total observation time. The results of phase 2 of the three-chamber sociability test experiment show that the mice in the blank control group have a certain percentage of resting time within the contact range of unfamiliar mouse 2, which is about 13.7%; that in the vehicle group is significantly lower, which is about 10.6%; and that in the plasminogen group is about 16.1%, which is significantly higher than in the vehicle group, and the statistical difference is close to significant (P=0.075) (Figure 12). It indicates that plasminogen can improve the social memory in multiple sclerosis model mice.

**Example 13 Plasminogen improves depressive behavior in multiple sclerosis model rats**

26 male rats at the age of 3 weeks are weighed and the abnormal rats are excluded according to weight, then all the rats are randomly divided into 2 groups, 8 rats in the blank control group and 18 rats in the model group. After the grouping is completed, rats in the blank control group are fed with normal maintenance diet (purchased from Beijing Keao Xieli Feed Co., Ltd), and rats in the model group are fed with a modeling diet containing 0.6% CPZ for 14 days to establish multiple sclerosis model [9]. Meanwhile all rats are started to be fed with normal maintenance diet. After completion of modeling, all rats are tested in the open field experiment, and the rats in the model group are grouped according to the test results, 7 rats in the vehicle group and 8 rats in the plasminogen group. After the grouping is completed, the administration to the rats in the vehicle group and the plasminogen group is started on day 1, and the rats in the plasminogen group are injected with plasminogen at 35 mg/kg through tail vein, and the rats in the vehicle group are injected with the vehicle at 3.5 ml/kg through tail vein, the treatment is lasted for 6 days. On the 7th day of administration, the open field experiment is performed.

The movement distance in the boundary zone is the length of the motion trail in the boundary zone during the test time of the open field experiment. The percentage of movement distance in the boundary zone = movement distance in the boundary zone / the sum of movement distance in the boundary zone and the central zone. The results show that the blank control group has a certain percentage of movement distance in the boundary zone, which is about 91.1%; that of the vehicle group increased significantly, which is about 93.6%; and that of the plasminogen group is about 88.1%, which is significantly lower than that of the vehicle group, and the statistical difference is significant (* indicates P<0.05) (Figure 13). It indicates that plasminogen can improve depressive behavior in multiple sclerosis model mice.

**Example 14 Plasminogen alleviates depressive behavior in multiple sclerosis model rats**

26 male rats at the age of 3 weeks are weighed and the abnormal rats are excluded according to weight, then all the rats are randomly divided into 2 groups, 8 rats in the blank control group and 18 rats in the model group. After the grouping is completed, rats in the blank control group are fed with normal maintenance diet (purchased from Beijing Keao Xieli Feed Co., Ltd), and rats in the model group are fed with a modeling diet containing 0.6% CPZ for 14 days to establish multiple sclerosis model [9]. Meanwhile all rats are started to be fed with normal maintenance diet. After completion of modeling, all rats are tested in the open field experiment, and the rats in the model group are grouped according to the test results, 7 rats in the vehicle group and 8 rats in the plasminogen group. After the grouping is completed, the administration to the rats in the vehicle group and the plasminogen group is started on day 1, and the rats in the plasminogen group are injected with plasminogen at 35 mg/kg through tail vein, and the rats in the vehicle group are injected with the vehicle at 3.5 ml/kg through tail vein, the treatment is lasted for 6 days, and no treatment to rats in the blank control group. On the 7th day of administration, the open field experiment is performed.

The movement distance in the central zone is the length of the motion trail in the central zone during the test time of the open field experiment. The percentage of movement distance in the central zone = the movement distance in the central zone/ the sum of the movement distance in the boundary zone and the central zone. The results show that the blank control group has a certain percentage of movement distance in the central zone, which is about 8.9%; the vehicle group has a significantly lower percentage, which is about 6.4%; the plasminogen group has about 11.9%, which is significantly higher than that of the vehicle group, and the statistical difference is significant (* indicates P<0.05) (Figure 14). It indicates that plasminogen can improve the anxious behavior of multiple sclerosis model rats.

**Example 15 Plasminogen alleviates anxious and depressive behavior in multiple sclerosis model mice**

30 female C57 mice are weighed before modeling, and after excluding the abnormal mice according to body weight, all mice are randomly divided into two groups, 8 mice in the blank control group and 22 mice in the model group. After grouping, mice in the blank control group are fed with normal maintenance diet (purchased from Beijing Keao Xieli Feed Co., Ltd), and mice in the model group are fed with modeling diet containing 0.6% bis(cyclohexanone) oxaldihydrazone (CPZ) (manufacturer: Shanghai Yuanye Bio-Technology Co., Ltd., item no. S30349) for 42 days to induce multiple sclerosis model [1]. After completion of modeling, all mice are tested in the open field experiment, and mice in the model group are grouped according to the test results, 11 mice in the vehicle group and 11 mice in the plasminogen group. After the grouping is completed, administration to all mice is started on day 1. The mice in the blank control group and the mice in the vehicle group are injected with the vehicle by tail vein injection at 0.1 ml/day per mouse, and the mice in the plasminogen group are injected with plasminogen by tail vein injection at 1 mg/day per mouse. The treatment is lasted for 20 days. During the administration period, all mice are fed with normal maintenance diet. An elevated cross-maze test is performed on day 21.

The elevated cross maze is used to examine the anxiety state of animals by using their exploratory nature of new and different environment and their fear of high open arms to form conflicting behaviors. The elevated cross maze has a pair of open arms and a pair of closed arms, and rodents tend to move in closed arms due to the darkness preference, but will move in the open arms out of curiosity and inquisitiveness. In the face of novel stimuli, animals have the urge to explore and fear at the same time, which causes the conflicting behaviors of exploration and avoidance, resulting in anxiety. While anxiolytic drugs can significantly increase the number and time of entering the open arm, the cross maze is higher from the ground, which is equivalent to the cliff that a person is standing, causing fear and anxiety in the experimental subjects. The elevated cross maze is widely used in the fields of new drug development/screening/evaluation, pharmacology, toxicology, preventive medicine, neurobiology, animal psychology and behavioral biology and other disciplines of science-research and computer-aided teaching, and is a classic experiment for medical schools and research institutions to conduct behavioral research, especially anxiety and depression research.

At the beginning of the test, the mice are placed into the maze from the central compartment and facing a closed arm and the activity is recorded for 5 minutes. Observation indicators include: number of entries of open arms (both front paws must enter the arm), dwell time in the open arms, number of entries of closed arms, and dwell time in closed arms. The proportion of dwell time in the open arms, the proportion of entries of open arms, and the total number of entries in the elevated cross maze are calculated. After the experiments are completed, the mice are removed, both arms are cleaned, and the odor is removed by spraying alcohol. Finally, the data are analyzed with ethological software.

Percentage of entries of open arms = total number of entries of open arms / total number of entries of open arms and closed arms. The results show that the mice in the blank control group have a certain percentage of entries of open arms, which is about 14.9%; the percentage of entries of open arms of the mice in the vehicle group is significantly increased, which is about 23.0%; the percentage of entries of open arms of the mice in the plasminogen group is about 14.5%, which is significantly less than that in the vehicle group, with a statistically significant difference (P=0.015) (Figure 15), and it is similar to that in the blank control group. It indicates that plasminogen can alleviate the anxious behavior of multiple sclerosis model mice to some extent.

**Example 16 Plasminogen alleviates anxious behavior in multiple sclerosis model mice**

30 female C57 mice are weighed before modeling, and after excluding abnormal mice according to body weight, all mice are randomly divided into two groups, 8 mice in the blank control group and 22 mice in the model group. After grouping, mice in the blank control group are fed with maintenance diet, and mice in the model group are fed with modeling diet containing 0.6% bis(cyclohexanone) oxaldihydrazone (CPZ) (manufacturer: Shanghai Yuanye Bio-Technology Co., Ltd., item no. S30349) for 42 days to induce multiple sclerosis model [1]. After the completion of modeling, all mice are tested in the open field experiment, and mice in the model group are grouped according to the test results, 11 mice in the vehicle group and 11 mice in the plasminogen group. After the grouping is completed, administration to all mice is started, and the start day is recorded as the first day of administration. The mice in the blank control group and the mice in the vehicle group are injected with the vehicle by tail vein injection at 0.1 ml/day per mouse, and the mice in the plasminogen group are injected with plasminogen by tail vein injection at 1 mg/day per mouse. The treatment is lasted for 20 days. During the administration period, all mice are fed with normal maintenance diet (purchased from Beijing Keao Xieli Feed Co., Ltd). An elevated-cross maze test is performed on day 21.

The percentage of entries of closed arms = total number of entries of closed arms/total number of entries of open and closed arms. The results show that the mice in the blank control group have a certain percentage of entries of closed arms, which is about 34.4%; the percentage of entries of closed arms of the mice in the vehicle group is significantly decreased, which is about 28.1%; the percentage of entries of closed arms of mice in the plasminogen group is about 37.1%, which is significantly greater than that in the vehicle group, with a statistically significant difference (P=0.007) (Figure 16), and it is similar to that in the blank control group. It indicates that plasminogen can alleviate the anxious and depressive behavior of multiple sclerosis model mice to some extent.

**Example 17 Plasminogen alleviates anxious and depressive behavior in multiple sclerosis model mice**

30 female C57 mice are weighed before modeling, and after excluding abnormal mice according to body weight, all mice are randomly divided into two groups, 8 mice in the blank control group and 22 mice in the model group. After grouping, mice in the blank control group are fed with normal maintenance diet (purchased from Beijing Keao Xieli Feed Co., Ltd), and mice in the model group are fed with modeling diet containing 0.6% bis(cyclohexanone) oxaldihydrazone (CPZ) (manufacturer: Shanghai Yuanye Bio-Technology Co., Ltd., item no. S30349) for 42 days to induce multiple sclerosis model [1]. After completion of modeling, all mice are tested in the open field experiment, and mice in the model group are grouped according to the test results, 11 mice in the vehicle group and 11 mice in the plasminogen group. After the grouping is completed, administration to all mice is started, and the start day is recorded as the first day of administration. The mice in the blank control group and the mice in the vehicle group are injected with the vehicle by tail vein injection at 0.1 ml/day per mouse, and the mice in the plasminogen group are injected with plasminogen by tail vein injection at 1 mg/day per mouse. The treatment is lasted for 27 days. During the administration period, all mice are fed with normal maintenance diet. An elevated cross-maze test is performed on day 28.

The total movement distance in closed arms is the total movement distance of mice in closed arms during the test time of the elevated cross-maze test. The results show that the mice in the blank control group have a certain total movement distance in closed arms, and the mice in the vehicle group have a significant increase in the total movement distance in closed arms. The total movement distance in closed arms of the mice in the plasminogen group is significantly less than that in the vehicle group, with statistically significant difference (* indicates $P<0.05$) (Figure 17). It indicates that plasminogen can alleviate the anxious and depressive behavior of multiple sclerosis model mice to some extent.

**Example 18 Plasminogen alleviates anxious and depressive behavior in multiple sclerosis model mice**

30 female C57 mice are weighed before modeling, and after excluding abnormal mice according to body weight, all mice are randomly divided into two groups, 8 mice in the blank control group and 22 mice in the model group. After grouping, mice in the blank control group are fed with normal maintenance diet (purchased from Beijing Keao Xieli Feed Co., Ltd), and mice in the model group are fed with modeling diet containing 0.6% bis(cyclohexanone) oxaldihydrazone (CPZ) (manufacturer: Shanghai Yuanye Bio-Technology Co., Ltd., item no. S30349) for 42 days to induce multiple sclerosis model [1]. After completion of modeling, all mice are tested in the open field experiment, and mice in the model group are grouped according to the test results, 11 mice in the vehicle group and 11 mice in the plasminogen group. After the grouping is completed, administration to all mice is started, and the start day is recorded as the first day of administration. The mice in the blank control group and the mice in the vehicle group are injected with the vehicle by tail vein injection at 0.1 ml/day per mouse, and the mice in the plasminogen group are injected with plasminogen by tail vein injection at 1 mg/day per mouse. The treatment is lasted for 27 days. During the administration period, all mice are fed with normal maintenance diet. An elevated cross-maze test is performed on day 28.

The percentage of resting time in closed arms = the resting time in closed arms/the sum of exercise time in closed arms and resting time in closed arms. The results show that the mice in the blank control group have a certain percentage of resting time in closed arms, which is about 51.8%; the percentage of resting time in closed arms of the mice in the vehicle group is significantly lower, which is about 36.8%; the percentage of resting time in closed arms of the mice in the plasminogen group is about 50.1%, which is significantly greater than that in the vehicle group, and the statistical difference is significant (* indicates $P<0.05$) (Figure 18) and it is similar to that in the blank control group. It indicates that plasminogen can alleviate the anxious and depressive behavior of multiple sclerosis model mice to some extent.

**Reference**

1. M. Lindner, S. Heine, K. Haastert, Sequential myelin protein expression during remyelination reveals fast and efficient repair after central nervous system demyelination, Neuropathology and Applied Neurobiology (2008), 34, 105-114.

2. Tuohy VK1, Lu Z, Sobel RA, Laursen RA et al. Identification of an encephalitogenic determinant of myelin proteolipid protein for SJL mice. J Immunol. 1989 Mar 1;142(5):1523-7.

3. Gotow T.Neurofilaments in health and disease. Med Electron Microsc. 2000;33(4):173-99.

4. Ahmed Z, Shaw G , Sharma V P , et al. Actin-binding Proteins Coronin-1a and IBA-1 Are Effective Microglial Markers for Immunohistochemistry[J]. Journal of Histochemistry and Cytochemistry, 2007, 55(7):687-700.

5. Kowianski, Przemys?aw, Lietzau G , Czuba E , et al. BDNF: A Key Factor with Multipotent Impact on Brain Signaling and Synaptic Plasticity[J]. Cellular & Molecular Neurobiology, 2017.

6. Hol E M, Pekny M. Glial fibrillary acidic protein (GFAP) and the astrocyte intermediate filament system in diseases of the central nervous system. [J]. Current Opinion in Cell Biology, 2015, 32(1):121-130.

7. Murphy R, O'Donoghue S, Counihan T, McDonald C, Calabresi PA, Ahmed MA, Kaplin A, Hallahan B. Neuropsychiatric syndromes of multiple sclerosis. J Neurol Neurosurg Psychiatry. 2017 Aug;88(8):697 -708.

8. Hirahara Y, Matsuda K I , Yamada H , et al. G protein-coupled receptor 30 contributes to improved remyelination after cuprizone-induced demyelination[J]. Glia, 2013, 61(3):420-431.

9. Di Biase A, Salvati S , Di Benedetto R , et al. Eicosapentaenoic acid pre-treatment reduces biochemical changes induced in total brain and myelin of weanling Wistar rats by cuprizone feeding[J]. Prostaglandins, Leukotrienes and Essential Fatty Acids (PLEFA), 2014, 90(4):99-104.58(1-2):40.

Sequence listing

SEQ ID NO: 1

gagcctctggatgactatgtgaatacccagggggcttcactgttcagtgtcactaagaagcagctgggagcaggaagtatagaagaatgtgcagcaaaatgtga ggaggacgaagaattcacctgcagggcattccaatatcacagtaaagagcaacaatgtgtgataatggctgaaaacaggaagtcctccataatcattaggatgagagat gtagttttatttgaaaagaaagtgtatctctcagagtgcaagactgggaatggaaagaactacagagggacgatgtccaaaacaaaaaatggcatcacctgtcaaaaatg gagttccacttctccccacagacctagattctcacctgctacacacccctcagagggactggaggagaactactgcaggaatccagacaacgatccgcaggggccctg gtgctatactactgatccagaaaagagatatgactactgcgacattcttgagtgtgaagaggaatgtatgcattgcagtggagaaaactatgacggcaaaatttccaagac catgtctggactggaatgccaggcctgggactctcagagcccacacgctcatggatacattccttccaaatttccaaacaagaacctgaagaagaattactgtcgtaacc ccgataggggagctgcggccttggtgtttcaccaccgaccccaacaagcgctgggaactttgtgacatcccccgctgcacaacacctccaccatcttctggtcccaccta ccagtgtctgaagggaacaggtgaaaactatcgcgggaatgtggctgttaccgtgtccgggcacacctgtcagcactggagtgcacagacccctcacacacataaca ggacaccagaaaacttcccctgcaaaaatttggatgaaaactactgccgcaatcctgacggaaaaagggccccatggtgccatacaaccaacagccaagtgcggtgg gagtactgtaagataccgtcctgtgactcctccccagtatccacggaacaattggctcccacagcaccacctgagctaacccctgtggtccaggactgctaccatggtga tggacagagctaccgaggcacatcctccaccaccaccacaggaaagaagtgtcagtcttggtcatctatgacaccacaccggcaccagaagacccccagaaaactacc caaatgctggcctgacaatgaactactgcaggaatccagatgccgataaaggcccctggtgtttaccacagaccccagcgtcaggtgggagtactgcaacctgaaaa aatgctcaggaacagaagcgagtgttgtagcacctccgcctgttgtcctgcttccagatgtagagactccttccgaagaagactgtatgtttgggaatgggaaaggatac cgaggcaagagggcgaccactgttactgggacgccatgccaggactgggctgcccaggagcccccatagacacagcattttcactccagagacaaatccacgggcg ggtctggaaaaaaattactgccgtaaccctgatggtgatgtaggtggtccctggtgctacacgacaaatccaagaaaactttacgactactgtgatgtccctcagtgtgcg gccccttcatttgattgtgggaagcctcaagtggagccgaagaaatgtcctggaagggttgtaggggggtgtgtggcccacccacattcctggccctggcaagtcagtc ttagaacaaggtttggaatgcacttctgtggaggcaccttgatatccccagagtgggtgttgactgctgcccactgcttggagaagtccccaaggccttcatcctacaagg tcatcctgggtgcacaccaagaagtgaatctcgaaccgcatgttcaggaaatagaagtgtctaggctgttcttggagcccacacgaaaagatattgccttgctaaagctaa gcagtcctgccgtcatcactgacaaagtaatcccagcttgtctgccatccccaaattatgtggtcgctgaccggaccgaatgtttcatcactggctggggagaaacccaa ggtactttggagctggccttctcaaggaagcccagctccctgtgattgagaataaagtgtgcaatcgctatgagtttctgaatggaagagtccaatccaccgaactctgtg ctgggcatttggccggaggcactgacagttgccagggtgacagtggaggtcctctggtttgcttcgagaaggacaaatacattttacaaggagtcacttcttggggtcttg gctgtgcacgccccaataagcctggtgtctatgttcgtgtttcaaggtttgttacttggattgagggagtgatgagaaataattaa

SEQ ID NO: 2

EPLDDYVNTQGASLFSVTKKQLGAGSIEECAAKCEEDEEFTCRAFQYHSKEQQCVIMAENRKSSIIR
MRDVVLFEKKVYLSECKTGNGKNYRGTMSKTKNGITCQKWSSTSPHRPRFSPATHPSEGLEENYCRNPDN
DPQGPWCYTTDPEKRYDYCDILECEEECMHCSGENYDGKISKTMSGLECQAWDSQSPHAGYIPSKFPNK

NLKKNYCRNPDRELRPWCFTTDPNKRWELCDIPRCTTPPPSSGPTYQCLKGTGENYRGNVAVTVSGHTCQ

HWSAQTPHTHNRTPENFPCKNLDENYCRNPDGKRAPWCHTTNSQVRWEYCKIPSCDSSPVSTEQLAPTAP

PELTPVVQDCYHGDGQSYRGTSSTTTTGKKCQSWSSMTPHRHQKTPENYPNAGLTMNYCRNPDADKGP

WCFTTDPSVRWEYCNLKKCSGTEASVVAPPPVVLLPDVETPSEEDCMFGNGKGYRGKRATTVTGTPCQD

WAAQEPHRHSIFTPETNPRAGLEKNYCRNPDGDVGGPWCYTTNPRKLYDYCDVPQCAAPSFDCGKPQVE

PKKCPGRVVGGCVAHPHSWPWQVSLRTRFGMHFCGGTLISPEWVLTAAHCLEKSPRPSSYKVILGAHQE

VNLEPHVQEIEVSRLFLEPTRKDIALLKLSSPAVITDKVIPACLPSPNYVVADRTECFITGWGETQGTFGAGL

LKEAQLPVIENKVCNRYEFLNGRVQSTELCAGHLAGGTDSCQGDSGGPLVCFEKDKYILQGVTSWGLGC

ARPNKPGVYVRVSRFVTWIEGVMRNN

SEQ ID NO: 3

atggaacataaggaagtggttcttctacttcttttatttctgaaatcaggtcaaggagagcctctggatgactatgtgaatacccagggggcttcactgttcagtgtca

ctaagaagcagctgggagcaggaagtatagaagaatgtgcagcaaaatgtgaggaggacgaagaattcacctgcagggcattccaatatcacagtaaagagcaaca

atgtgtgataatggctgaaaacaggaagtcctccataatcattaggatgagagatgtagtttttatttgaaaagaaagtgtatctctcagagtgcaagactgggaatggaaag

aactacagagggacgatgtccaaaacaaaaaatggcatcacctgtcaaaaatggagttccacttctccccacagacctagattctcacctgctacacacccctcagagg

gactggaggagaactactgcaggaatccagacaacgatccgcaggggccctggtgctatactactgatccagaaaagagatatgactactgcgacattcttgagtgtga

agaggaatgtatgcattgcagtggagaaaactatgacggcaaaattccaagaccatgtctggactggaatgccaggcctgggactctcagagcccacacgctcatgg

atacattccttccaaatttccaaacaagaacctgaagaagaattactgtcgtaaccccgatagggagctgcggccttggtgtttcaccaccgaccccaacaagcgctggg

aactttgtgacatcccccgctgcacaacacctccaccatcttctggtcccacctaccagtgtctgaagggaacaggtgaaaactatcgcgggaatgtggctgttaccgtgt

ccgggcacacctgtcagcactggagtgcacagacccctcacacacataacaggacaccagaaaacttccctgcaaaaatttggatgaaaactactgccgcaatcctg

acggaaaaagggccccatggtgccatacaaccaacagccaagtgcggtggggagtactgtaagataccgtcctgtgactcctccccagtatccacggaacaattggctc

ccacagcaccacctgagctaacccctgtggtccaggactgctaccatggtgatggacagagctaccgaggcacatcctccaccaccaccacaggaaagaagtgtcag

tcttggtcatctatgacaccacaccggcaccagaagacccccagaaaactacccaaatgctggcctgacaatgaactactgcaggaatccagatgccgataaaggcccc

tggtgttttaccacagaccccagcgtcaggtgggagtactgcaacctgaaaaaatgctcaggaacagaagcgagtgttgtagcacctccgcctgttgtcctgcttccaga

tgtagagactccttccgaagaagactgtatgtttgggaatgggaaaggataccgaggcaagagggcgaccactgttactgggacgccatgccaggactgggctgccc

aggagcccccatagacacagcattttcactccagagacaaatccacgggcgggtctggaaaaaaattactgccgtaaccctgatggtgatgtaggtggtccctggtgcta

cacgacaaatccaagaaaactttacgactactgtgatgtccctcagtgtgcggcccccttcatttgattgtgggaagcctcaagtggagccgaagaaatgtcctggaaggg

ttgtaggggggtgtgtggcccacccacattcctggccctggcaagtcagtcttagaacaaggtttggaatgcacttctgtggaggcaccttgatatccccagagtgggtgt

tgactgctgcccactgcttggagaagtccccaaggccttcatcctacaaggtcatcctgggtgcacaccaagaagtgaatctcgaaccgcatgttcaggaaatagaagt

gtctaggctgttcttggagcccacacgaaaagatattgccttgctaaagctaagcagtcctgccgtcatcactgacaaagtaatcccagcttgtctgccatccccaaattat

gtggtcgctgaccggaccgaatgtttcatcactggctggggagaaacccaaggtacttttggagctggccttctcaaggaagcccagctccctgtgattgagaataaagt

gtgcaatcgctatgagtttctgaatggaagagtccaatccaccgaactctgtgctgggcatttggccggaggcactgacagttgccagggtgacagtggaggtcctctg

gtttgcttcgagaaggacaaatacattttacaaggagtcacttcttggggtcttggctgtgcacgccccaataagcctggtgtctatgttcgtgtttcaaggtttgttacttgga

ttgagggagtgatgagaaataattaa

SEQ ID NO: 4

MEHKEVVLLLLLLFLKSGQGEPLDDYVNTQGASLFSVTKKQLGAGSIEECAAKCEEDEEFTCRAFQYH

SKEQQCVIMAENRKSSIIIRMRDVVLFEKKVYLSECKTGNGKNYRGTMSKTKNGITCQKWSSTSPHRPRFS

PATHPSEGLEENYCRNPDNDPQGPWCYTTDPEKRYDYCDILECEEECMHCSGENYDGKISKTMSGLECQA

WDSQSPHAHGYIPSKFPNKNLKKNYCRNPDRELRPWCFTTDPNKRWELCDIPRCTTPPPSSGPTYQCLKGT

GENYRGNVAVTVSGHTCQHWSAQTPHTHNRTPENFPCKNLDENYCRNPDGKRAPWCHTTNSQVRWEYC

KIPSCDSSPVSTEQLAPTAPPELTPVVQDCYHGDGQSYRGTSSTTTTGKKCQSWSSMTPHRHQKTPENYPN

AGLTMNYCRNPDADKGPWCFTTDPSVRWEYCNLKKCSGTEASVVAPPPVVLLPDVETPSEEDCMFGNGK

GYRGKRATTVTGTPCQDWAAQEPHRHSIFTPETNPRAGLEKNYCRNPDGDVGGPWCYTTNPRKLYDYCD

VPQCAAPSFDCGKPQVEPKKCPGRVVGGCVAHPHSWPWQVSLRTRFGMHFCGGTLISPEWVLTAAHCLE

KSPRPSSYKVILGAHQEVNLEPHVQEIEVSRLFLEPTRKDIALLKLSSPAVITDKVIPACLPSPNYVVADRTE

CFITGWGETQGTFGAGLLKEAQLPVIENKVCNRYEFLNGRVQSTELCAGHLAGGTDSCQGDSGGPLVCFE

KDKYILQGVTSWGLGCARPNKPGVYVRVSRFVTWIEGVMRNN

SEQ ID NO: 5

aaagtgtatctctcagagtgcaagactgggaatggaaagaactacagagggacgatgtccaaaacaaaaaatggcatcacctgtcaaaaatggagttccacttc

tccccacagacctagattctcacctgctacacaccccctcagagggactggaggagaactactgcaggaatccagacaacgatccgcaggggccctggtgctatactac

tgatccagaaaagagatatgactactgcgacattcttgagtgtgaagaggaatgtatgcattgcagtggagaaaactatgacggcaaaatttccaagaccatgtctggact

ggaatgccaggcctgggactctcagagcccacacgctcatggatacattccttccaaatttccaaacaagaacctgaagaagaattactgtcgtaaccccgatagggag

ctgcggccttggtgtttcaccaccgaccccaacaagcgctgggaactttgtgacatcccccgctgcacaacacctccaccatcttctggtcccacctaccagtgtctgaa

gggaacaggtgaaaactatcgcgggaatgtggctgttaccgtgtccgggcacacctgtcagcactggagtgcacagacccctcacacacataacaggacaccagaa

aacttcccctgcaaaaatttggatgaaaactactgccgcaatcctgacggaaaaagggccccatggtgccatacaaccaacagccaagtgcggtgggagtactgtaag

ataccgtcctgtgactcctccccagtatccacggaacaattggctcccacagcaccacctgagctaacccctgtggtccaggactgctaccatggtgatggacagagct

accgaggcacatcctccaccaccaccacaggaaagaagtgtcagtcttggtcatctatgacaccacaccggcaccagaagaccccagaaaactacccaaatgctggc

ctgacaatgaactactgcaggaatccagatgccgataaaggcccctggtgttttaccacagaccccagcgtcaggtgggagtactgcaacctgaaaaaatgctcagga

acagaagcgagtgttgtagcacctccgcctgttgtcctgcttccagatgtagagactccttccgaagaagactgtatgtttgggaatgggaaaggataccgaggcaaga

gggcgaccactgttactgggacgccatgccaggactgggctgcccaggagcccccatagacacagcattttcactccagagacaaatccacgggcgggtctggaaaa

aaattactgccgtaaccctgatggtgatgtaggtggtccctggtgctacacgacaaatccaagaaaactttacgactactgtgatgtccctcagtgtgcggccccttcattt

gattgtgggaagcctcaagtggagccgaagaaatgtcctggaagggttgtagggggtgtgtggcccacccacattcctggccctggcaagtcagtcttagaacaagg

tttggaatgcacttctgtggaggcaccttgatatccccagagtgggtgttgactgctgcccactgcttggagaagtccccaaggccttcatcctacaaggtcatcctgggt

gcacaccaagaagtgaatctcgaaccgcatgttcaggaaatagaagtgtctaggctgttcttggagcccacacgaaaagatattgccttgctaaagctaagcagtcctgc

cgtcatcactgacaaagtaatcccagcttgtctgccatccccaaattatgtggtcgctgaccggaccgaatgtttcatcactggctggggagaaacccaaggtacttttgg

agctggccttctcaaggaagcccagctccctgtgattgagaataaagtgtgcaatcgctatgagtttctgaatggaagagtccaatccaccgaactctgtgctgggcattt

ggccggaggcactgacagttgccagggtgacagtggaggtcctctggtttgcttcgagaaggacaaatacattttacaaggagtcacttcttggggtcttggctgtgcac

gccccaataagcctggtgtctatgttcgtgtttcaaggtttgttacttggattgagggagtgatgagaaataattaa

SEQ ID NO: 6

KVYLSECKTGNGKNYRGTMSKTKNGITCQKWSSTSPHRPRFSPATHPSEGLEENYCRNPDNDPQGPW

CYTTDPEKRYDYCDILECEEECMHCSGENYDGKISKTMSGLECQAWDSQSPHAHGYIPSKFPNKNLKKNY

CRNPDRELRPWCFTTDPNKRWELCDIPRCTTPPPSSGPTYQCLKGTGENYRGNVAVTVSGHTCQHWSAQT

PHTHNRTPENFPCKNLDENYCRNPDGKRAPWCHTTNSQVRWEYCKIPSCDSSPVSTEQLAPTAPPELTPVV

QDCYHGDGQSYRGTSSTTTTGKKCQSWSSMTPHRHQKTPENYPNAGLTMNYCRNPDADKGPWCFTTDP

SVRWEYCNLKKCSGTEASVVAPPPVVLLPDVETPSEEDCMFGNGKGYRGKRATTVTGTPCQDWAAQEPH

RHSIFTPETNPRAGLEKNYCRNPDGDVGGPWCYTTNPRKLYDYCDVPQCAAPSFDCGKPQVEPKKCPGR

VVGGCVAHPHSWPWQVSLRTRFGMHFCGGTLISPEWVLTAAHCLEKSPRPSSYKVILGAHQEVNLEPHV

QEIEVSRLFLEPTRKDIALLKLSSPAVITDKVIPACLPSPNYVVADRTECFITGWGETQGTFGAGLLKEAQLP

VIENKVCNRYEFLNGRVQSTELCAGHLAGGTDSCQGDSGGPLVCFEKDKYILQGVTSWGLGCARPNKPG

VYVRVSRFVTWIEGVMRNN

SEQ ID NO: 7

gagcctctggatgactatgtgaatacccaggggggcttcactgttcagtgtcactaagaagcagctgggagcaggaagtatagaagaatgtgcagcaaaatgtga

ggaggacgaagaattcacctgcagggcattccaatatcacagtaaagagcaacaatgtgtgataatggctgaaaacaggaagtcctccataatcattaggatgagagat

gtagtttatttgaaaagaaagtgtatctctcagagtgcaagactgggaatggaaagaactacagagggacgatgtccaaaacaaaaaatggcatcacctgtcaaaaatg

gagttccacttctccccacagacctagattctcacctgctacacacccctcagagggactggaggagaactactgcaggaatccagacaacgatccgcaggggccctg

gtgctatactactgatccagaaaagagatatgactactgcgacattcttgagtgtgaagaggcggcccccttcatttgattgtgggaagcctcaagtggagccgaagaaat

gtcctggaagggttgtagggggtgtgtggcccacccacattcctggccctggcaagtcagtcttagaacaaggtttggaatgcacttctgtggaggcaccttgatatcc

ccagagtgggtgttgactgctgcccactgcttggagaagtccccaaggccttcatcctacaaggtcatcctgggtgcacaccaagaagtgaatctcgaaccgcatgttca

ggaaatagaagtgtctaggctgttcttggagcccacacgaaaagatattgccttgctaaagctaagcagtcctgccgtcatcactgacaaagtaatcccagcttgtctgcc

atccccaaattatgtggtcgctgaccggaccgaatgtttcatcactggctggggagaaacccaaggtacttttggagctggccttctcaaggaagcccagctccctgtgat

tgagaataaagtgtgcaatcgctatgagtttctgaatggaagagtccaatccaccgaactctgtgctgggcatttggccggaggcactgacagttgccagggtgacagtg

gaggtcctctggtttgcttcgagaaggacaaatacattttacaaggagtcacttcttggggtcttggctgtgcacgccccaataagcctggtgtctatgttcgtgtttcaaggt

ttgttacttggattgagggagtgatgagaaataattaa

SEQ ID NO: 8

EPLDDYVNTQGASLFSVTKKQLGAGSIEECAAKCEEDEEFTCRAFQYHSKEQQCVIMAENRKSSIIR
MRDVVLFEKKVYLSECKTGNGKNYRGTMSKTKNGITCQKWSSTSPHRPRFSPATHPSEGLEENYCRNPDN
DPQGPWCYTTDPEKRYDYCDILECEEAAPSFDCGKPQVEPKKCPGRVVGGCVAHPHSWPWQVSLRTRFG
MHFCGGTLISPEWVLTAAHCLEKSPRPSSYKVILGAHQEVNLEPHVQEIEVSRLFLEPTRKDIALLKLSSPA
VITDKVIPACLPSPNYVVADRTECFITGWGETQGTFGAGLLKEAQLPVIENKVCNRYEFLNGRVQSTELCA
GHLAGGTDSCQGDSGGPLVCFEKDKYILQGVTSWGLGCARPNKPGVYVRVSRFVTWIEGVMRNN

SEQ ID NO: 9

gtcaggtggggagtactgcaacctgaaaaaatgctcaggaacagaagcgagtgttgtagcacctccgcctgttgtcctgcttccagatgtagagactccttccgaa
gaagactgtatgtttgggaatgggaaaggataccgaggcaagagggcgaccactgttactgggacgccatgccaggactgggctgcccaggagccccatagacaca
gcattttcactccagagacaaatccacgggcgggtctggaaaaaaattactgccgtaaccctgatggtgatgtaggtggtccctggtgctacacgacaaatccaagaaa
actttacgactactgtgatgtccctcagtgtgcggccccttcatttgattgtgggaagcctcaagtggagccgaagaaatgtcctggaaggggttgtagggggggtgtgtggc
ccacccacattcctggccctggcaagtcagtcttagaacaaggtttggaatgcacttctgtggaggcaccttgatatccccagagtgggtgttgactgctgcccactgctt
ggagaagtccccaaggccttcatcctacaaggtcatcctgggtgcacaccaagaagtgaatctcgaaccgcatgttcaggaaatagaagtgtctaggctgttcttggag
cccacacgaaaagatattgccttgctaaagctaagcagtcctgccgtcatcactgacaaagtaatcccagcttgtctgccatccccaaattatgtggtcgctgaccggacc
gaatgtttcatcactggctggggagaaacccaaggtacttttggagctggccttctcaaggaagcccagctccctgtgattgagaataaagtgtgcaatcgctatgagtttc
tgaatggaagagtccaatccaccgaactctgtgctgggcatttggccggaggcactgacagttgccagggtgacagtggaggtcctctggtttgcttcgagaaggacaa
atacattttacaaggagtcacttcttggggtcttggctgtgcacgccccaataagcctggtgtctatgttcgtgtttcaaggtttgttacttggattgagggagtgatgagaaat
aattaa

SEQ ID NO: 10

VRWEYCNLKKCSGTEASVVAPPPVVLLPDVETPSEEDCMFGNGKGYRGKRATTVTGTPCQDWAAQ
EPHRHSIFTPETNPRAGLEKNYCRNPDGDVGGPWCYTTNPRKLYDYCDVPQCAAPSFDCGKPQVEPKKCP
GRVVGGCVAHPHSWPWQVSLRTRFGMHFCGGTLISPEWVLTAAHCLEKSPRPSSYKVILGAHQEVNLEP
HVQEIEVSRLFLEPTRKDIALLKLSSPAVITDKVIPACLPSPNYVVADRTECFITGWGETQGTFGAGLLKEA
QLPVIENKVCNRYEFLNGRVQSTELCAGHLAGGTDSCQGDSGGPLVCFEKDKYILQGVTSWGLGCARPN
KPGVYVRVSRFVTWIEGVMRNN

SEQ ID NO: 11

gccccttcatttgattgtgggaagcctcaagtggagccgaagaaatgtcctggaagggttgtaggggggtgtgtggcccacccacattcctggccctggcaagt

cagtcttagaacaaggtttggaatgcacttctgtggaggcaccttgatatccccagagtgggtgttgactgctgcccactgcttggagaagtccccaaggccttcatccta

caaggtcatcctgggtgcacaccaagaagtgaatctcgaaccgcatgttcaggaaatagaagtgtctaggctgttcttggagcccacacgaaaagatattgccttgctaa

agctaagcagtcctgccgtcatcactgacaaagtaatcccagcttgtctgccatccccaaattatgtggtcgctgaccggaccgaatgtttcatcactggctggggagaaa

cccaaggtacttttggagctggccttctcaaggaagcccagctccctgtgattgagaataaagtgtgcaatcgctatgagtttctgaatggaagagtccaatccaccgaac

tctgtgctgggcatttggccggaggcactgacagttgccagggtgacagtggaggtcctctggtttgcttcgagaaggacaaatacattttacaaggagtcacttcttggg

gtcttggctgtgcacgccccaataagcctggtgtctatgttcgtgtttcaaggtttgttacttggattgagggagtgatgagaaataattaa

SEQ ID NO: 12

APSFDCGKPQVEPKKCPGRVVGGCVAHPHSWPWQVSLRTRFGMHFCGGTLISPEWVLTAAHCLEKS
PRPSSYKVILGAHQEVNLEPHVQEIEVSRLFLEPTRKDIALLKLSSPAVITDKVIPACLPSPNYVVADRTECFI
TGWGETQGTFGAGLLKEAQLPVIENKVCNRYEFLNGRVQSTELCAGHLAGGTDSCQGDSGGPLVCFEKD
KYILQGVTSWGLGCARPNKPGVYVRVSRFVTWIEGVMRNN

SEQ ID NO: 13

gttgtaggggggtgtgtggcccacccacattcctggccctggcaagtcagtcttagaacaaggtttggaatgcacttctgtggaggcaccttgatatccccagagt
gggtgttgactgctgcccactgcttggagaagtccccaaggccttcatcctacaaggtcatcctgggtgcacaccaagaagtgaatctcgaaccgcatgttcaggaaata
gaagtgtctaggctgttcttggagcccacacgaaaagatattgccttgctaaagctaagcagtcctgccgtcatcactgacaaagtaatcccagcttgtctgccatcccca
aattatgtggtcgctgaccggaccgaatgtttcatcactggctggggagaaacccaaggtacttttggagctggccttctcaaggaagcccagctccctgtgattgagaat
aaagtgtgcaatcgctatgagtttctgaatggaagagtccaatccaccgaactctgtgctgggcatttggccggaggcactgacagttgccagggtgacagtggaggtc
ctctggtttgcttcgagaaggacaaatacattttacaaggagtcacttcttggggtcttggctgtgcacgccccaataagcctggtgtctatgttcgtgtttcaaggtttgttac
ttggattgagggagtgatgaga

SEQ ID NO: 14

VVGGCVAHPHSWPWQVSLRTRFGMHFCGGTLISPEWVLTAAHCLEKSPRPSSYKVILGAHQEVNLEP
HVQEIEVSRLFLEPTRKDIALLKLSSPAVITDKVIPACLPSPNYVVADRTECFITGWGETQGTFGAGLLKEA
QLPVIENKVCNRYEFLNGRVQSTELCAGHLAGGTDSCQGDSGGPLVCFEKDKYILQGVTSWGLGCARPN
KPGVYVRVSRFVTWIEGVMR

**Claims**

1. A method for preventing and treating multiple sclerosis comprising: administering to a subject a therapeutically effective amount of one or more compounds selected from the group consisting of: a component of plasminogen activation pathway, a compound directly activating plasminogen or indirectly activating plasminogen by activating an upstream component of a plasminogen activation pathway, a compound mimicking the activity of plasminogen or plasmin, a compound up-regulating the expression of plasminogen or an activator of plasminogen, an analog of plasminogen, an analog of plasmin, an analog of tPA or uPA and an antagonist of fibrinolysis inhibitor.

2. The method according to claim 1, wherein the component of plasminogen activation pathway is selected from the group consisting of: plasminogen, recombinant human plasmin, Lys-plasminogen, Glu-plasminogen, plasmin, a variant or an analog of plasminogen or plasmin comprising one or more kringle domains or protease domains of plasminogen and plasmin, mini-plasminogen, mini-plasmin, micro-plasminogen, micro-plasmin, delta-plasminogen, delta-plasmin, an activator of plasminogen, tPA and uPA.

3. The method according to claim 1, wherein the antagonist of the fibrinolysis inhibitor is an inhibitor of PAI-1, complement C1 inhibitor, α2 antiplasmin or α2 macroglobulin, e.g., an antibody.

4. The method according to any one of claims 1-3, wherein the compound has one or more activities selected from the group consisting of: promoting regeneration of nerve myelin sheath, promoting the expression of myelin protein, promoting the expression of NFP in nerve tissue, promoting regeneration of nerve fiber, promoting the expression of NFP in nerve tissue, increasing the level of MBP in nerve tissue, increasing the number of microglia in nerve tissue, promoting repair of nerve tissue inflammation, promoting the activity of astrocyte in nerve tissue, increasing the level of BDNF in nerve tissue, promoting the expression of GFAP in nerve tissue, improving the social behavior ability of the subject, improving the social memory ability of the subject, alleviating the depressive behavior of the subject, alleviating the anxious behavior of the subject.

5. The method according to any one of claims 1-4, wherein the compound is plasminogen.

6. The method according to any one of claims 1-5, wherein the plasminogen has at least 75%, 80%, 85%, 90%, 95%, 96%, 97%, 98% or 99% sequence identity with SEQ ID NO: 2 and has the proteolytic activity of plasminogen.

7. The method according to any one of claims 1-6, wherein the plasminogen has at least 75%, 80%, 85%, 90%, 95%, 96%, 97%, 98%, or 99% sequence identity with SEQ ID NO: 2 and has the lysine binding activity of plasminogen.

8. The method according to any one of claims 1-6, wherein the plasminogen comprises an amino acid sequence having at least 75%, 80%, 85%, 90%, 95%, 96%, 97%, 98% or 99% sequence identity with the active fragment of plasminogen represented by SEQ ID NO: 14, and has the proteolytic activity of plasminogen.

9. The method according to any one of claims 1-6, wherein the plasminogen is a conservative substitution variant of the plasminogen of SEQ ID NO: 2.

10. The method according to any one of claims 1-6, wherein the plasminogen is natural or synthetic human plasminogen.

11. The method according to any one of claims 1-10, wherein the compound is used in combination with one or more other therapeutic methods or medicaments.

12. The method according to claim 11, wherein the other therapeutic methods are one or more selected from the group consisting of: surgical treatments, cell therapies (including stem cell therapies), and physical therapies.

13. The method according to claim 11, wherein the other medicaments are one or more selected from the group consisting of: hormone, immunosuppressant, neurotrophic medicament, antibiotic, and antiviral medicament.

14. The method according to any one of claims 1-13, wherein the compound is administered by any one or more means or routes selected from the group consisting of: nasal inhalation, aerosol inhalation, nasal drop, eye drop, ear drop, intravenous administration, intraperitoneal administration, subcutaneous administration, intracranial administration, intrathecal administration, intraarterial administration and intramuscular administration.

Figure 1

Figure 2

Figure 3

Figure 4

Figure 5

Figure 6

Figure 7

Figure 8

Figure 9

Figure 10

Figure 11

Figure 12

Figure 13

Figure 14

Figure 15

Figure 16

Figure 17

Figure 18

## INTERNATIONAL SEARCH REPORT

| International application No. |
|---|
| **PCT/CN2021/075921** |

**A.    CLASSIFICATION OF SUBJECT MATTER**

A61K 38/43(2006.01)i

According to International Patent Classification (IPC) or to both national classification and IPC

**B.    FIELDS SEARCHED**

Minimum documentation searched (classification system followed by classification symbols)

A61K

Documentation searched other than minimum documentation to the extent that such documents are included in the fields searched

Electronic data base consulted during the international search (name of data base and, where practicable, search terms used)

Genbank, EMBL, VEN, CNKI, CNABS, CNTXT, WOTXT, USTXT, EPTXT, JPTXT, PubMed, ISI Web of Knowledge: plasminogen, Multiple, sclerosis, tPA, uPA, 纤维蛋白溶酶原激活, 纤溶酶原, 多发性硬化症

**C.    DOCUMENTS CONSIDERED TO BE RELEVANT**

| Category* | Citation of document, with indication, where appropriate, of the relevant passages | Relevant to claim No. |
|---|---|---|
| X | WO 0124815 A1 (HUMAN GENOME SCIENCES, INC.) 12 April 2001 (2001-04-12)<br>    the abstract, and page 114, lines 23-30 | 1 |
| Y | WO 0124815 A1 (HUMAN GENOME SCIENCES, INC.) 12 April 2001 (2001-04-12)<br>    the abstract, and page 2, paragraphs 2-4, and page 114, lines 23-30 | 1-14 |
| Y | ZEA, M. et al. "Relationship between Fibrinolytic System and Neurological Diseases"<br>*Reviews Neurology*, Vol. 51, No. 5, 01 September 2010 (2010-09-01),<br>    page 298, right column, paragraph 2 | 1-14 |
| Y | FORSGREN, M. et al. "Plasminogen[Homo sapiens]"<br>*GenBank: NP_000292.1*, 19 March 1999 (1999-03-19),<br>    entire document | 6-14 |
| A | WO 2012107567 A2 (OXPROTECT GMBH) 15 November 2012 (2012-11-15)<br>    entire document | 1-14 |
| A | CN 108210910 A (TALENGEN INSTITUTE OF LIFE SCIENCES, CO. LTD.) 29 June 2018<br>(2018-06-29)<br>    entire document | 1-14 |

☑ Further documents are listed in the continuation of Box C.          ☑ See patent family annex.

| | |
|---|---|
| *    Special categories of cited documents:<br>"A"    document defining the general state of the art which is not considered to be of particular relevance<br>"E"    earlier application or patent but published on or after the international filing date<br>"L"    document which may throw doubts on priority claim(s) or which is cited to establish the publication date of another citation or other special reason (as specified)<br>"O"    document referring to an oral disclosure, use, exhibition or other means<br>"P"    document published prior to the international filing date but later than the priority date claimed | "T"    later document published after the international filing date or priority date and not in conflict with the application but cited to understand the principle or theory underlying the invention<br>"X"    document of particular relevance; the claimed invention cannot be considered novel or cannot be considered to involve an inventive step when the document is taken alone<br>"Y"    document of particular relevance; the claimed invention cannot be considered to involve an inventive step when the document is combined with one or more other such documents, such combination being obvious to a person skilled in the art<br>"&"    document member of the same patent family |

| Date of the actual completion of the international search | Date of mailing of the international search report |
|---|---|
| **17 April 2021** | **07 May 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

## INTERNATIONAL SEARCH REPORT

| | International application No. |
|---|---|
| | **PCT/CN2021/075921** |

### C.    DOCUMENTS CONSIDERED TO BE RELEVANT

| Category* | Citation of document, with indication, where appropriate, of the relevant passages | Relevant to claim No. |
|---|---|---|
| A | TEESALU, T. et al. "Tissue Plasminogen Activator as a Key Effector in Neurobiology and Neuropathology" *Biochemical Society Transactions,* Vol. 30, No. 2, 30 April 2002 (2002-04-30), pp. 183-189 | 1-14 |
| A | CUZNER, M. L. et al. "Plasminogen Activators and Matrix Metalloproteases, Mediators of Extracellular Proteolysis in Inflammatory Demyelination of the Central Nervous System" *Journal of Neuroimmunology,* Vol. 94, No. 1-2, 01 February 1999 (1999-02-01), pp. 1-14 | 1-14 |
| A | SHAW, M. A. et al. "Plasminogen Deficiency Delays the Onset and Protects from Demyelination and Paralysis in Autoimmune Neuroinflammatory DiseaseJ Neurosci" *Journal of Neuroscience,* Vol. 37, No. 14, 08 March 2017 (2017-03-08), pp. 3776-3788 | 1-14 |

Form PCT/ISA/210 (second sheet) (January 2015)

**INTERNATIONAL SEARCH REPORT**

| International application No. |
| --- |
| **PCT/CN2021/075921** |

**Box No. I        Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  ☑  forming part of the international application as filed:

        ☑  in the form of an Annex C/ST.25 text file.

        ☐  on paper or in the form of an image file.

    b.  ☐  furnished together with the international application under PCT Rule 13*ter*.1(a) for the purposes of international search only in the form of an Annex C/ST.25 text file.

    c.  ☐  furnished subsequent to the international filing date for the purposes of international search only:

        ☐  in the form of an Annex C/ST.25 text file (Rule 13*ter*.1(a)).

        ☐  on paper or in the form of an image file (Rule 13*ter*.1(b) and Administrative Instructions, Section 713).

2.  ☐  In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (January 2015)

**INTERNATIONAL SEARCH REPORT**

| International application No. |
|---|
| **PCT/CN2021/075921** |

**Box No. II       Observations where certain claims were found unsearchable (Continuation of item 2 of first sheet)**

This international search report has not been established in respect of certain claims under Article 17(2)(a) for the following reasons:

1. ☑   Claims Nos.: **1-14**
   because they relate to subject matter not required to be searched by this Authority, namely:

   [1]   Claims 1-14 relate to a method for the treatment or diagnosis of a living human or animal body,
   and therefore do not warrant an international search according to the criteria set out in PCT Rule
   39.1(iv). This search report is made on the basis of "the application of the preparation of drugs for the
   prevention and treatment of multiple sclerosis".

2. ☐   Claims Nos.:
   because they relate to parts of the international application that do not comply with the prescribed requirements to such an
   extent that no meaningful international search can be carried out, specifically:

3. ☐   Claims Nos.:
   because they are dependent claims and are not drafted in accordance with the second and third sentences of Rule 6.4(a).

Form PCT/ISA/210 (continuation of first sheet) (January 2015)

<div align="center">

**INTERNATIONAL SEARCH REPORT**
**Information on patent family members**

</div>

International application No.

**PCT/CN2021/075921**

| Patent document cited in search report | | | Publication date (day/month/year) | Patent family member(s) | | | Publication date (day/month/year) |
|---|---|---|---|---|---|---|---|
| WO | 0124815 | A1 | 12 April 2001 | US | 2002094955 | A1 | 18 July 2002 |
| | | | | CA | 2385703 | A1 | 12 April 2001 |
| | | | | US | 2003180934 | A1 | 25 September 2003 |
| | | | | EP | 1227833 | A1 | 07 August 2002 |
| | | | | JP | 2003524413 | A | 19 August 2003 |
| | | | | AU | 1191001 | A | 10 May 2001 |
| WO | 2012107567 | A2 | 15 November 2012 | DE | 102011003944 | A1 | 16 August 2012 |
| | | | | US | 10416170 | B2 | 17 September 2019 |
| | | | | EP | 2673640 | A2 | 18 December 2013 |
| | | | | US | 9316650 | B2 | 19 April 2016 |
| | | | | WO | 2012107567 | A3 | 15 November 2012 |
| | | | | US | 2014044796 | A1 | 13 February 2014 |
| | | | | US | 2016208235 | A1 | 21 July 2016 |
| | | | | EP | 2673640 | B1 | 30 September 2020 |
| CN | 108210910 | A | 29 June 2018 | CN | 108210899 | A | 29 June 2018 |
| | | | | HK | 1257584 | A1 | 25 October 2019 |
| | | | | HK | 1257585 | A1 | 25 October 2019 |
| | | | | CN | 108210905 | A | 29 June 2018 |
| | | | | HK | 1257587 | A1 | 25 October 2019 |
| | | | | HK | 1257588 | A1 | 25 October 2019 |
| | | | | HK | 1257589 | A1 | 25 October 2019 |
| | | | | CN | 108210897 | A | 29 June 2018 |
| | | | | CN | 108210892 | A | 29 June 2018 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 102154253 A
- WO 9704801 A
- US 3773919 A
- EP 58481 A

### Non-patent literature cited in the description

- **NY, A. ; LEONARDSSON, G. ; HAGGLUND, A.C ; HAGGLOF, P. ; PLOPLIS, V.A. ; CARMELIET, P. ; NY, T.** Ovulation inplasminogen-deficient mice. *Endocrinology,* 1999, vol. 140, 5030-5035
- **SILVERSTEIN RL ; LEUNG LL ; HARPEL PC ; NACHMAN RL.** Complex formation of platelet thrombospondin with plasminogen. Modulation of activation by tissue activator. *J. Clin. Invest.,* November 1984, vol. 74 (5), 1625-33
- **GRAVANIS I ; TSIRKA SE.** Tissue-type plasminogen activator as a therapeutic target in stroke. *Expert Opinion on Therapeutic Targets,* February 2008, vol. 12 (2), 159-70
- **GEIGER M ; HUBER K ; WOJTA J ; STINGL L ; ESPANA F ; GRIFFIN JH ; BINDER BR.** Complex formation between urokinase and plasma protein C inhibitor in vitro and in vivo. *Blood,* August 1989, vol. 74 (2), 722-8
- **AISINA RB ; MUKHAMETOVA L I.** Structure and function of plasminogen/plasmin system [J. *Russian Journal of Bioorganic Chemistry,* 2014, vol. 40 (6), 590-605
- **BARANY.** Solid-Phase Peptide Synthesis. *The Peptides: Analysis, Synthesis, Biology,* vol. 2, 3-284
- **MERRIFIELD et al.** Special Methods in Peptide Synthesis. *J. Am. Chem. Soc.,* 1963, vol. 85, 2149-2156
- **STEWART et al.** Solid Phase Peptide Synthesis. Pierce Chem. Co, 1984
- **GANESAN A.** *Mini Rev. Med Chem.,* 2006, vol. 6, 3-10
- **CAMARERO JA et al.** *Protein Pept Lett.,* 2005, vol. 12, 723-8
- **WINNACKER.** From Genes to Clones. VCH Publishers, 1987
- **CO et al.** *J. Immunol.,* 1992, vol. 148, 1149
- Remington's Pharmaceutical Sciences. 1980
- **LANGER et al.** *J. Biomed. Mater. Res.,* 1981, vol. 15, 167-277
- **LANGER.** *Chem. Tech.,* 1982, vol. 12, 98-105
- **KENNETH C ROBBINS ; LOUIS SUMMARIA ; DAVID ELWYN et al.** Further Studies on the Purification and Characterization of Human Plasminogen and Plasmin. *Journal of Biological Chemistry,* 1965, vol. 240 (1), 541-550
- **SUMMARIA L ; SPITZ F ; ARZADON L et al.** Isolation and characterization of the affinity chromatography forms of human Glu-and Lys-plasminogens and plasmins. *J Biol Chem.,* 25 June 1976, vol. 251 (12), 3693-9
- **HAGAN JJ ; ABLONDI FB ; DE RENZO EC.** Purification and biochemical properties of human plasminogen. *J Biol Chem.,* April 1960, vol. 235, 1005-10
- **M. LINDNER ; S. HEINE ; K. HAASTERT.** Sequential myelin protein expression during remyelination reveals fast and efficient repair after central nervous system demyelination. *Neuropathology and Applied Neurobiology,* 2008, vol. 34, 105-114
- **TUOHY VK1 ; LU Z ; SOBEL RA ; LAURSEN RA et al.** Identification of an encephalitogenic determinant of myelin proteolipid protein for SJL mice. *J Immunol.,* 01 March 1989, vol. 142 (5), 1523-7
- **GOTOW T.** Neurofilaments in health and disease. *Med Electron Microsc,* 2000, vol. 33 (4), 173-99
- **AHMED Z ; SHAW G ; SHARMA V P et al.** Actin-binding Proteins Coronin-1a and IBA-1 Are Effective Microglial Markers for Immunohistochemistry[J. *Journal of Histochemistry and Cytochemistry,* 2007, vol. 55 (7), 687-700
- **KOWIANSKI ; PRZEMYS?AW ; LIETZAU G ; CZUBA E et al.** BDNF: A Key Factor with Multipotent Impact on Brain Signaling and Synaptic Plasticity[J. *Cellular & Molecular Neurobiology,* 2017
- **HOL E M ; PEKNY M.** Glial fibrillary acidic protein (GFAP) and the astrocyte intermediate filament system in diseases of the central nervous system. *J]. Current Opinion in Cell Biology,* 2015, vol. 32 (1), 121-130

- **MURPHY R ; O'DONOGHUE S ; COUNIHAN T ; MCDONALD C ; CALABRESI PA ; AHMED MA ; KAPLIN A ; HALLAHAN B.** Neuropsychiatric syndromes of multiple sclerosis. *J Neurol Neurosurg Psychiatry,* August 2017, vol. 88 (8), 697-708
- **HIRAHARA Y ; MATSUDA K I ; YAMADA H et al.** G protein-coupled receptor 30 contributes to improved remyelination after cuprizone-induced demyelination[J. *Glia,* 2013, vol. 61 (3), 420-431

- **DI BIASE A ; SALVATI S ; DI BENEDETTO R et al.** Eicosapentaenoic acid pre-treatment reduces biochemical changes induced in total brain and myelin of weanling Wistar rats by cuprizone feeding[J. *Prostaglandins, Leukotrienes and Essential Fatty Acids (PLEFA),* 2014, vol. 90,58 (4,1-2), 99-104, 40